# EUROPEAN PATENT SPECIFICATION

(11) **EP 4 615 386 B1**
(45) Date of publication and mention of the grant of the patent: **07.10.2026**
(21) Application number: 23808869.4
(22) Date of filing: 09.11.2023
(51) Int. Cl.: A61F 13/0246, A61F 13/05

(54) **A WOUND DRESSING**
WUNDVERBAND
PANSEMENT

(30) Priority: 10.11.2022 US 202263424163 P; 22.12.2022 GB 202219610
(43) Date of publication of application: 17.09.2025
(73) Proprietor: ConvaTec Limited, Deeside, Flintshire CH5 2NU (GB)
(72) Inventor: BROWN, Natalie, Deeside, Flintshire CH5 2NU (GB); KESTEVEN, Donna, Deeside, Flintshire CH5 2NU (GB)
(74) Representative: Wilson Gunn
(86) International application number: PCT/GB2023/052925
(87) International publication number: WO 2024/100403

(56) References cited:
- EP-A1- 3 915 531
- WO-A1-2020/104426
- WO-A1-2021/214203
- US-A1- 2014 309 574
- US-A1- 2019 231 602
- US-A1- 2021 236 342

## Description

### Technical Field of the Invention

The present invention relates to a wound dressing. **In** particular, the invention concerns a negative pressure wound dressing.

### Background to the Invention

Wound dressings are known and are generally suitable for treating a variety of wounds, including chronic and acute wound types, such as infected wounds, venous ulcers, diabetic ulcers, burns and surgical wounds.

Negative pressure has been used to treat a range of chronic and acute wounds. Negative pressure may facilitate wound healing through a number of mechanisms, including removal of excess exudate, reduction in periwound edema and increased perfusion. Combined with the physical forces exerted by th negative pressure which draw the wound edges together, this can result in improved wound outcomes.

Wound dressings typically comprise an adhesive skin contact layer for detachably adhering the dressing to a dermal surface. Conventionally, the adhesive skin contact layer comprises a plurality of perforations (i.e., through holes in the adhesive skin contact layer) which enable fluid, for example wound exudate, to flow through the layer but which prevent tissue ingrowth into other material comprised in the wound dressing. Typically, the perforations have a diameter of no more than 1.2mm to facilitate adhesion of the skin contact layer to the dermal surface and to provide a skin contact layer with sufficient breathability, i.e., allowing water vapour and gas to pass through the layer. Typically, the skin contact layer comprises a silicone adhesive. Disadvantageously, some of the perforations are prone to closing, or at least reducing in diameter, after formation due to the silicone adhesive entering and, therefore, blocking the perforations. While this may increase adhesion of the layer to the dermal surface, this adversely affects the breathability of the skin contact layer, causing an accumulation of bodily fluid at and in proximity to the skin or wound site, therefore, preventing the formation of an environment optimised for wound healing. Conversely, it is known to increase the size of perforations in an adhesive skin contact layer to aid breathability. However, this is achieved at the expense of adhesion of the skin contact layer to a dermal surface and in such cases, the skin contact layer may only weakly adhere to a dermal surface such that movement of a patient while the wound dressing is adhered may cause the wound dressing to partially or completely detach from the patient, adversely affecting the dressing to transmit a negative pressure to the wound site.

Conventional techniques to form the perforations in the adhesive layer include using a hot pin process. However, disadvantageously, this results in the formation of 'slugs' around the perforation (i.e., the material intended to be removed to form the perforation is not wholly disconnected from the main body of material and, therefore, partially obstructs the perforation and results in a non-planar surface of the skin contact layer). To overcome this, ultrasound may be used to create the perforations. However, this process disadvantageously forms doughnut/volcano structure around the perforations.

Portable systems have been developed which include a means to manage the exudate produced by the wound by collecting exudate within the wound dressing, typically in an absorbent material, and by evaporation through the dressing. Such systems mean that a separate collection canister may not be an essential part of the system. An advantage of not needing a canister is that the device is less bulky and more portable. A disadvantage with such devices is that if the dressing exceeds its fluid handling capacity, exudate may be drawn from the absorbent material(s) and enter the pump. The presence of exudate in the pump will eventually cause it to fail and require its replacement. The therapy provided by the system may also be less than optimal due to the potential for excess exudate to collect, or pool, at the wound interface. **In** order to prevent fouling of the pump with exudate, it is known to provide a barrier layer between the absorbent material and the pump. However, th liquid barrier layer does not improve the ability of the absorbent material(s) to absorb exudate.

Wound contact layers are known to comprise a mixture of gelling and non-gelling fibres. Gelling fibres form a gel upon contact with exudate. However, upon forming a gel, many wound contact layers weaken and in some cases partially dissociate from other layers comprised in an absorbent structure. To overcome this, it is known to provide non-gelling fibres as part of the wound contact surface of the wound contact layer. Such arrangements aim to strike a balance between providing a sufficient amount of gelling fibres and preventing dissociation of the wound contact layer whn it becomes wet with exudate.

However, known wound contact layers fail to adequately strike this balance and such layers either do not adequately prevent pooling of exudate at the wound contact surface or do not adequately adhere to other layers within the wound dressing when the wound contact layer becomes wet with exudate.

During negative pressure therapy, air as well as wound exudate material is removed from a wound site. The wound exudate must be collected remotely from the wound site. Some known negative pressure therapy systems comprise a waste cannister connected to a pump unit for the collection and storage of wound exudate material. However, the use of a canister can result in the therapy system itself being bulky and expensive to manufacture. Also, replacing a canister or a container in a canister in whch wound exudate is collected can be a time consuming and a relatively unhygienic process.

Negative pressure wound dressings have been produced which comprise an absorbent layer to absorb wound exudate in an attempt to remove the need for a cannister and pump system. However, such wound dressings often fail to sufficiently meet two requirements of an absorbent layer in a wound dressing, i.e., that the absorbent layer has a high absorbance capacity and remains flexible (i.e., does not stiffen) when it absorbs wound exudate which subsequently dries.

Known absorbent layers with a relatively low absorbance capacity cause pooling of wound exudate at the wound site. This is both uncomfortable and unhygienic for the patient and for a healthcare professional who replaces the wound dressing. Moreover, there is a risk that the wound and/or surrounding skin will become macerated if in prolonged contact with excess wound exudate.

Moreover, known absorbent layers often become relatively stiff when wound exudate has been absorbed and subsequently dried. This results in the fabric of the absorbent layer 'doming' which, disadvantageously, provides a tunnel effect of air transfer and causes air to leak from the wound dressing, thereby adversely affecting the negative pressure transmitted to the wound site.

Conventional negative pressure wound dressings often comprise relatively rigid dressing and binding components which adversely affects system utility and which provides a relatively rigid wound dressing which is disadvantageous in respect of user comfort. Moreover, such components require individual manufacturing steps to incorporate thm into the wound dressing, therefore, increasing the time of manufacture and cost.

The purpose of such components is to attempt to increase the integrity of the wound dressing to prevent, or at least reduce, dissociation of one or more of the layers comprised therein. However, the advantages of such components often do not outweigh the disadvantages associated with the additional manufacturing steps, complex processing and the rigidity of the final wound dressing. Further, the rigid dressing and binding components often have an adverse effect on the wicking, or movement, of wound exudate through the wound dressing away from the wound site. Additionally, existing negative pressure wound dressing systems do not utilise absorbent materials and additional wound dressing components arranged to maximise the management of wound exudate within the dressing.

It is known to provide a wound dressing comprising a wound contact layer, a transmission layer and an absorbent layer, arranged such that the transmission layer overlies the wound contact layer, and the absorbent layer overlies the transmission layer. This arrangement is disclosed in US 10,507,141. However, such an arrangement does not provide a significant absorbency capacity. This means that the arrangement suffers from unnecessary pooling of wound exudate at the wound site. As such, the hygiene of the wound dressing and of the wound site could be improved which in turn would increase comfort for the user.

US 2021/236342 A1 describes a negative pressure wound dressing comprising a gas occlusive layer which may be coupled to the skin surrounding a wound, and a patient interface layer configured to contact the wound and/or the skin surrounding the wound. A sorbent layer may be formed from a superabsorbent polymer or a superabsorbent wound care laminate, and which may be provided between the gas occlusive layer and the patient interface layer.

US 2019/231602 describes a negative pressure wound dressing comprising a plurality of layers, including a gel layer for providing a fluid seal to a tissue site. The dressing comprises a cover having an attachment device such as a pressure sensitive adhesive to bond the cover to the skin surrounding the tissue site. The cover may be coated with an adhesive coating having a weight of 25-65 gsm.

It is an object of embodiments of th present invention to at least partially overcome or alleviate the above problems and/or to provide an improved wound dressing.

### Summary of the Invention

According to a first aspect of the present invention there is provided a negative pressure wound dressing, the dressing comprising a backing layer, an adhesive skin contact layer and an absorbent structure arranged between the backing layer and the adhesive skin contact layer, wherein the adhesive skin contact layer is configured to detachably adhere the dressing to a dermal surface, wherein the backing layer comprises a coupling member configured to connect the dressing to a negative pressure source, wherein the adhesive skin contact layer has a weight of between 40gsm and 250gsm, further wherein the adhesive skin contact layer is a perforated mesh comprising perforations each having a diameter of between 1.25mm and 5.00mm.

The adhesive skin contact layer comprises perforations each having a diameter of between 1.25mm and 5.00mm. Advantageously, this means that the perforations have a sufficient diameter to provide breathability to the wound dressing in the event that some of the perforations reduce in diameter after their formation. As such, the adhesive skin contact layer maintains an environment optimised for wound healing.

It has surprisingly been found that the relatively large perforations, compared to the perforations known in the prior art having a diameter of 1.20mm, did not adversely affect adhesion to the dermal surface. Thus, advantageously, the invention functions without loss, or at least significant loss, of adhesion of the wound dressing to the dermal surface and, therefore, reliably transmits a negative pressure to the wound site.

Moreover, it has been found that following manufacture of the adhesive skin contact layer, perforations having a diameter of between 1.25mm and 5.00mm do not 'close-up', or at least do not substantially close-up. Comparatively, perforations having a smaller diameter, for example 1.20mm, exhibit a tendency to close-up after formation due to the silicone adhesive entering and, therefore, blocking the perforations. Thus, the present invention provides for an adhesive skin contact layer which ensures sufficient breathability to the wound dressing, in use.

The perforations comprise through holes in the adhesive skin contact layer which enable fluid to flow through the layer. Thus, in use, if the adhesive skin contact layer is arranged covering and in contact with a wound, the adhesive skin contact layer prevents, or at least substantially prevents, tissue ingrowth into other materials of the wound dressing.

Beneficially, the adhesive skin contact layer helps to maintain the integrity of the wound dressing while also creating an airtight, or substantially airtight, seal around the absorbent structure in order to maintain negative pressure at the wound.

The absorbent structure may be arranged between the backing layer and the adhesive skin contact layer. Advantageously, this means that wound exudate is absorbed by the absorbent structure before the exudate contacts the backing layer.

The adhesive skin contact layer may have a weight of about 40gsm, about 50gsm, about 60gsm, about 70gsm, about 80gsm, about 90gsm, about 100gsm, about 110gsm, about 120gsm, about 130gsm, about 140gsm, about 150gsm, about 155gsm, about 160gsm, about 165gsm, about 170gsm, about 175gsm, about 180gsm, about 185gsm, about 190gsm, about 195gsm, about 200gsm, about 205gsm, about 210gsm, about 215gsm, about 220gsm, about 225gsm, about 230gsm, about 235gsm, about 240gsm, about 245gsm, or about 250gsm.

The adhesive skin contact layer may have a weight of at least about 40gsm, about 50gsm, about 60gsm, about 70gsm, about 80gsm, about 90gsm, about 100gsm, about 110gsm, about 120gsm, about 130gsm, about 140gsm, about 150gsm, about 155gsm, about 160gsm, about 165gsm, about 170gsm, about 175gsm, about 180gsm, about 185gsm, about 190gsm, about 195gsm, about 200gsm, about 205gsm, about 210gsm, about 215gsm, about 220gsm, about 225gsm, about 230gsm, about 235gsm, about 240gsm, about 245gsm, or at least about 250gsm.

The adhesive skin contact layer may have a weight of no more than about 40gsm, about 50gsm, about 60gsm, about 70gsm, about 80gsm, about 90gsm, about 100gsm, about 110gsm, about 120gsm, about 130gsm, about 140gsm, about 150gsm, about 155gsm, about 160gsm, about 165gsm, about 170gsm, about 175gsm, about 180gsm, about 185gsm, about 190gsm, about 195gsm, about 200gsm, about 205gsm, about 210gsm, about 215gsm, about 220gsm, about 225gsm, about 230gsm, about 235gsm, about 240gsm, about 245gsm, or no more than about 250gsm.

The adhesive skin contact layer may have a weight of between about 40gsm and about 250gsm, about 50gsm and about 250gsm, about 60gsm and about 250gsm, about 70gsm and about 250gsm, about 80gsm and about 250gsm, about 90gsm and about 250gsm, about 100gsm and about 250gsm, 125gsm and about 245gsm, about 135gsm and about 240gsm, about 150gsm and about 235gsm, about 160gsm and about 230gsm, about 170gsm and about 230gsm, about 175gsm and about 225gsm, about 180gsm and about 220gsm, about 185gsm and about 215gsm, about 190gsm and about 210gsm, about 195gsm and bout 205gsm, or about 200gsm.

The adhesive skin contact layer may have a weight of between about 40 gsm and about 250 gsm, between about 40 gsm and about 240 gsm, between about 40 gsm and about 230 gsm, between about 40 gsm and about 220 gsm, between about 40 gsm and about 210 gsm, between about 40 gsm and about 200 gsm, between about 40 gsm and about 190 gsm, between about 40 gsm and about 180 gsm, between about 40 gsm and about 170 gsm, between about 40 gsm and about 160 gsm, between about 40 gsm and about 150 gsm, between about 40 gsm and about 140 gsm, between about 40 gsm and about 130 gsm, between about 40 gsm and about 120 gsm, between about 40 gsm and about 110 gsm, between about 40 gsm and about 100 gsm, between about 40 gsm and about 90 gsm, between about 40 gsm and about 80 gsm, between about 40 gsm and about 70 gsm, between about 40 gsm and about 60 gsm, or between about 40 gsm and about 50 gsm.

The adhesive skin contact layer may have a weight of between about 50 gsm and about 250 gsm, between about 50 gsm and about 240 gsm, between about 50 gsm and about 230 gsm, between about 50 gsm and about 220 gsm, between about 50 gsm and about 210 gsm, between about 50 gsm and about 200 gsm, between about 50 gsm and about 190 gsm, between about 50 gsm and about 180 gsm, between about 50 gsm and about 170 gsm, between about 50 gsm and about 160 gsm, between about 50 gsm and about 150 gsm, between about 50 gsm and about 140 gsm, between about 50 gsm and about 130 gsm, between about 50 gsm and about 120 gsm, between about 50 gsm and about 110 gsm, between about 50 gsm and about 100 gsm, between about 50 gsm and about 90 gsm, between about 50 gsm and about 80 gsm, between about 50 gsm and about 70 gsm, or between about 50 gsm and about 60 gsm.

The adhesive skin contact layer may have a weight of between about 60 gsm and about 250 gsm, between about 60 gsm and about 240 gsm, between about 60 gsm and about 230 gsm, between about 60 gsm and about 220 gsm, between about 60 gsm and about 210 gsm, between about 60 gsm and about 200 gsm, between about 60 gsm and about 190 gsm, between about 60 gsm and about 180 gsm, between about 60 gsm and about 170 gsm, between about 60 gsm and about 160 gsm, between about 60 gsm and about 150 gsm, between about 60 gsm and about 140 gsm, between about 60 gsm and about 130 gsm, between about 60 gsm and about 120 gsm, between about 60 gsm and about 110 gsm, between about 60 gsm and about 100 gsm, between about 60 gsm and about 90 gsm, between about 60 gsm and about 80 gsm, or between about 60 gsm and about 70 gsm.

The adhesive skin contact layer may have a weight of between about 70 gsm and about 250 gsm, between about 70 gsm and about 240 gsm, between about 70 gsm and about 230 gsm, between about 70 gsm and about 220 gsm, between about 70 gsm and about 210 gsm, between about 70 gsm and about 200 gsm, between about 70 gsm and about 190 gsm, between about 70 gsm and about 180 gsm, between about 70 gsm and about 170 gsm, between about 70 gsm and about 160 gsm, between about 70 gsm and about 150 gsm, between about 70 gsm and about 140 gsm, between about 70 gsm and about 130 gsm, between about 70 gsm and about 120 gsm, between about 70 gsm and about 110 gsm, between about 70 gsm and about 100 gsm, between about 70 gsm and about 90 gsm, or between about 70 gsm and about 80 gsm.

The adhesive skin contact layer may have a weight of between about 80 gsm and about 250 gsm, between about 80 gsm and about 240 gsm, between about 80 gsm and about 230 gsm, between about 80 gsm and about 220 gsm, between about 80 gsm and about 210 gsm, between about 80 gsm and about 200 gsm, between about 80 gsm and about 190 gsm, between about 80 gsm and about 180 gsm, between about 80 gsm and about 170 gsm, between about 80 gsm and about 160 gsm, between about 80 gsm and about 150 gsm, between about 80 gsm and about 140 gsm, between about 80 gsm and about 130 gsm, between about 80 gsm and about 120 gsm, between about 80 gsm and about 110 gsm, between about 80 gsm and about 100 gsm, or between about 80 gsm and about 90 gsm.

The adhesive skin contact layer may have a weight of between about 90 gsm and about 250 gsm, between about 90 gsm and about 240 gsm, between about 90 gsm and about 230 gsm, between about 90 gsm and about 220 gsm, between about 90 gsm and about 210 gsm, between about 90 gsm and about 200 gsm, between about 90 gsm and about 190 gsm, between about 90 gsm and about 180 gsm, between about 90 gsm and about 170 gsm, between about 90 gsm and about 160 gsm, between about 90 gsm and about 150 gsm, between about 90 gsm and about 140 gsm, between about 90 gsm and about 130 gsm, between about 90 gsm and about 120 gsm, between about 90 gsm and about 110 gsm, or between about 90 gsm and about 100 gsm.

The adhesive skin contact layer may have a weight of between about 100 gsm and about 250 gsm, between about 100 gsm and about 240 gsm, between about 100 gsm and about 230 gsm, between about 100 gsm and about 220 gsm, between about 100 gsm and about 210 gsm, between about 100 gsm and about 200 gsm, between about 100 gsm and about 190 gsm, between about 100 gsm and about 180 gsm, between about 100 gsm and about 170 gsm, between about 100 gsm and about 160 gsm, between about 100 gsm and about 150 gsm, between about 100 gsm and about 140 gsm, between about 100 gsm and about 130 gsm, between about 100 gsm and about 120 gsm, or between about 100 gsm and about 110 gsm.

The adhesive skin contact layer may have a weight of between about 110 gsm and about 250 gsm, between about 110 gsm and about 240 gsm, between about 110 gsm and about 230 gsm, between about 110 gsm and about 220 gsm, between about 110 gsm and about 210 gsm, between about 110 gsm and about 200 gsm, between about 110 gsm and about 190 gsm, between about 110 gsm and about 180 gsm, between about 110 gsm and about 170 gsm, between about 110 gsm and about 160 gsm, between about 110 gsm and about 150 gsm, between about 110 gsm and about 140 gsm, between about 110 gsm and about 130 gsm, or between about 110 gsm and about 120 gsm.

The adhesive skin contact layer may have a weight of between about 120 gsm and about 250 gsm, between about 120 gsm and about 240 gsm, between about 120 gsm and about 230 gsm, between about 120 gsm and about 220 gsm, between about 120 gsm and about 210 gsm, between about 120 gsm and about 200 gsm, between about 120 gsm and about 190 gsm, between about 120 gsm and about 180 gsm, between about 120 gsm and about 170 gsm, between about 120 gsm and about 160 gsm, between about 120 gsm and about 150 gsm, between about 120 gsm and about 140 gsm, or between about 120 gsm and about 130 gsm.

The adhesive skin contact layer may have a weight of between about 130 gsm and about 250 gsm, between about 130 gsm and about 240 gsm, between about 130 gsm and about 230 gsm, between about 130 gsm and about 220 gsm, between about 130 gsm and about 210 gsm, between about 130 gsm and about 200 gsm, between about 130 gsm and about 190 gsm, between about 130 gsm and about 180 gsm, between about 130 gsm and about 170 gsm, between about 130 gsm and about 160 gsm, between about 130 gsm and about 150 gsm, or between about 130 gsm and about 140 gsm.

The adhesive skin contact layer may have a weight of between about 140 gsm and about 250 gsm, between about 140 gsm and about 240 gsm, between about 140 gsm and about 230 gsm, between about 140 gsm and about 220 gsm, between about 140 gsm and about 210 gsm, between about 140 gsm and about 200 gsm, between about 140 gsm and about 190 gsm, between about 140 gsm and about 180 gsm, between about 140 gsm and about 170 gsm, between about 140 gsm and about 160 gsm, or between about 140 gsm and about 150 gsm.

The adhesive skin contact layer may have a weight of between about 150 gsm and about 250 gsm, between about 150 gsm and about 240 gsm, between about 150 gsm and about 230 gsm, between about 150 gsm and about 220 gsm, between about 150 gsm and about 210 gsm, between about 150 gsm and about 200 gsm, between about 150 gsm and about 190 gsm, between about 150 gsm and about 180 gsm, between about 150 gsm and about 170 gsm, or between about 150 gsm and about 160 gsm.

The adhesive skin contact layer may have a weight of between about 160 gsm and about 250 gsm, between about 160 gsm and about 240 gsm, between about 160 gsm and about 230 gsm, between about 160 gsm and about 220 gsm, between about 160 gsm and about 210 gsm, between about 160 gsm and about 200 gsm, between about 160 gsm and about 190 gsm, between about 160 gsm and about 180 gsm, or between about 160 gsm and about 170 gsm.

The adhesive skin contact layer may have a weight of between about 170 gsm and about 250 gsm, between about 170 gsm and about 240 gsm, between about 170 gsm and about 230 gsm, between about 170 gsm and about 220 gsm, between about 170 gsm and about 210 gsm, between about 170 gsm and about 200 gsm, between about 170 gsm and about 190 gsm, or between about 170 gsm and about 180 gsm.

The adhesive skin contact layer may have a weight of between about 180 gsm and about 250 gsm, between about 180 gsm and about 240 gsm, between about 180 gsm and about 230 gsm, between about 180 gsm and about 220 gsm, between about 180 gsm and about 210 gsm, between about 180 gsm and about 200 gsm, or between about 180 gsm and about 190 gsm.

The adhesive skin contact layer may have a weight of between about 190 gsm and about 250 gsm, between about 190 gsm and about 240 gsm, between about 190 gsm and about 230 gsm, between about 190 gsm and about 220 gsm, between about 190 gsm and about 210 gsm, or between about 190 gsm and about 200 gsm.

The adhesive skin contact layer may have a weight of between about 200 gsm and about 250 gsm, between about 200 gsm and about 240 gsm, between about 200 gsm and about 230 gsm, between about 200 gsm and about 220 gsm, or between about 200 gsm and about 210 gsm.

The adhesive skin contact layer may have a weight of between about 210 gsm and about 250 gsm, between about 210 gsm and about 240 gsm, between about 210 gsm and about 230 gsm, or between about 210 gsm and about 220 gsm.

The adhesive skin contact layer may have a weight of between about 220 gsm and about 250 gsm, between about 220 gsm and about 240 gsm, or between about 220 gsm and about 230 gsm.

The adhesive skin contact layer may have a weight of between about 230 gsm and about 250 gsm, or between about 230 gsm and about 240 gsm.

The adhesive skin contact layer may have a weight of between about 240 gsm and about 250 gsm.

Advantageously, an adhesive skin contact layer having a weight of between about 100gsm and about 250gsm provides a more malleable, gelled structure so, in the event of an air leak, the layer can seal the leak more easily than if the layer had a weight below 100gsm, in particular below 40gsm. Further, adhesive skin contact layers having a weight greater than 250gsm exhibit processing problems, for example the adhesive may 'flow out' from a cut shape, rendering the adhesive skin contact layer unusable.

The adhesive skin contact layer may comprise perforations each having a diameter of about 1.25mm, about 1.30mm, about 1.35mm, about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.10mm, about 3.20mm, about 3.30mm, about 3.40mm, about 3.50mm, about 3.60mm, about 3.70mm, about 3.80mm, about 3.90mm, about 4.00mm, about 4.10mm, about 4.20mm, about 4.30mm, about 4.40mm, about 4.50mm, about 4.60mm, about 4.70mm, about 4.80mm, about 4.90mm, or about 5.00mm.

The adhesive skin contact layer may comprise perforations each having a diameter of at least about 1.25mm, about 1.30mm, about 1.35mm, about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.10mm, about 3.20mm, about 3.30mm, about 3.40mm, about 3.50mm, about 3.60mm, about 3.70mm, about 3.80mm, about 3.90mm, about 4.00mm, about 4.10mm, about 4.20mm, about 4.30mm, about 4.40mm, about 4.50mm, about 4.60mm, about 4.70mm, about 4.80mm, about 4.90mm, or at least about 5.00mm.

The adhesive skin contact layer may comprise perforations each having a diameter of no more than about 1.25mm, about 1.30mm, about 1.35mm, about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.10mm, about 3.20mm, about 3.30mm, about 3.40mm, about 3.50mm, about 3.60mm, about 3.70mm, about 3.80mm, about 3.90mm, about 4.00mm, about 4.10mm, about 4.20mm, about 4.30mm, about 4.40mm, about 4.50mm, about 4.60mm, about 4.70mm, about 4.80mm, about 4.90mm, or no more than about 5.00mm.

The adhesive skin contact layer may comprise perforations each having a diameter of between about 1.25mm and about 5.00mm, about 1.50mm and about 4.50mm, about 1.75mm and about 4.00mm, about 1.90mm and about 3.50mm, about 2.00mm and about 3.00mm, about 2.05mm and about 2.80mm, about 2.10mm and about 2.60mm, about 2.15mm and about 2.50mm, about 2.18mm and about 2.40mm, about 2.19mm and about 2.30mm, or about 2.20mm.

The adhesive skin contact layer may comprise perforations each having a diameter of between about 1.75mm and about 4.00mm, about 2.00mm and about 3.50mm, about 2.10mm and about 3.00mm, about 2.15mm and about 2.50mm, or about 2.20mm.

The adhesive skin contact layer may comprise perforations each having a diameter of between about 1.25mm and about 5.00mm, about 1.25mm and about 4.95mm, about 1.25mm and about 4.90mm, about 1.25mm and about 4.80 mm, about 1.25mm and about 4.70 mm, about 1.25mm and about 4.60mm, about 1.25mm and about 4.50 mm, about 1.25mm and about 4.40 mm, about 1.25mm and about 4.30 mm, about 1.25mm and about 4.20 mm, about 1.25mm and about 4.10 mm, about 1.25mm and about 4.00 mm, about 1.25mm and about 3.90 mm, about 1.25mm and about 3.80 mm, about 1.25mm and about 3.70 mm, about 1.25mm and about 3.60 mm, about 1.25mm and about 3.50 mm, about 1.25mm and about 3.40 mm, about 1.25mm and about 3.30 mm, about 1.25mm and about 3.20 mm, about 1.25mm and about 3.10 mm, about 1.25mm and about 3.00 mm, about 1.25mm and about 2.90 mm, about 1.25mm and about 2.80 mm, about 1.25mm and about 2.70 mm, about 1.25mm and about 2.60 mm, about 1.25mm and about 2.50 mm, about 1.25mm and about 2.40 mm, about 1.25mm and about 2.30 mm, about 1.25mm and about 2.20 mm, about 1.25mm and about 2.10 mm, about 1.25mm and about 2.00 mm, about 1.25mm and about 1.90 mm, about 1.25mm and about 1.80 mm, about 1.25mm and about 1.70 mm, about 1.25mm and about 1.60 mm, about 1.25mm and about 1.50 mm, about 1.25mm and about 1.40 mm, or between about 1.25mm and about 1.30 mm.

The adhesive skin contact layer may comprise perforations each having a diameter of between about 1.30mm and about 5.00mm, about 1.30mm and about 4.95mm, about 1.30mm and about 4.90mm, about 1.30mm and about 4.80 mm, about 1.30mm and about 4.70 mm, about 1.30mm and about 4.60mm, about 1.30mm and about 4.50 mm, about 1.30mm and about 4.40 mm, about 1.30mm and about 4.30 mm, about 1.30mm and about 4.20 mm, about 1.30mm and about 4.10 mm, about 1.30mm and about 4.00 mm, about 1.30mm and about 3.90 mm, about 1.30mm and about 3.80 mm, about 1.30mm and about 3.70 mm, about 1.30mm and about 3.60 mm, about 1.30mm and about 3.50 mm, about 1.30mm and about 3.40 mm, about 1.30mm and about 3.30 mm, about 1.30mm and about 3.20 mm, about 1.30mm and about 3.10 mm, about 1.30mm and about 3.00 mm, about 1.30mm and about 2.90 mm, about 1.30mm and about 2.80 mm, about 1.30mm and about 2.70 mm, about 1.30mm and about 2.60 mm, about 1.30mm and about 2.50 mm, about 1.30mm and about 2.40 mm, about 1.30mm and about 2.30 mm, about 1.30mm and about 2.20 mm, about 1.30mm and about 2.10 mm, about 1.30mm and about 2.00 mm, about 1.30mm and about 1.90 mm, about 1.30mm and about 1.80 mm, about 1.30mm and about 1.70 mm, about 1.30mm and about 1.60 mm, about 1.30mm and about 1.50 mm, or between about 1.30mm and about 1.40 mm.

The adhesive skin contact layer may comprise perforations each having a diameter of between about 1.40mm and about 5.00mm, about 1.40mm and about 4.95mm, about 1.40mm and about 4.90mm, about 1.40mm and about 4.80 mm, about 1.40mm and about 4.70 mm, about 1.40mm and about 4.60mm, about 1.40mm and about 4.50 mm, about 1.40mm and about 4.40 mm, about 1.40mm and about 4.30 mm, about 1.40mm and about 4.20 mm, about 1.40mm and about 4.10 mm, about 1.40mm and about 4.00 mm, about 1.40mm and about 3.90 mm, about 1.40mm and about 3.80 mm, about 1.40mm and about 3.70 mm, about 1.40mm and about 3.60 mm, about 1.40mm and about 3.50 mm, about 1.40mm and about 3.40 mm, about 1.40mm and about 3.30 mm, about 1.40mm and about 3.20 mm, about 1.40mm and about 3.10 mm, about 1.40mm and about 3.00 mm, about 1.40mm and about 2.90 mm, about 1.40mm and about 2.80 mm, about 1.40mm and about 2.70 mm, about 1.40mm and about 2.60 mm, about 1.40mm and about 2.50 mm, about 1.40mm and about 2.40 mm, about 1.40mm and about 2.30 mm, about 1.40mm and about 2.20 mm, about 1.40mm and about 2.10 mm, about 1.40mm and about 2.00 mm, about 1.40mm and about 1.90 mm, about 1.40mm and about 1.80 mm, about 1.40mm and about 1.70 mm, about 1.40mm and about 1.60 mm, or between about 1.40mm and about 1.50 mm.

The adhesive skin contact layer may comprise perforations each having a diameter of between about 1.50mm and about 5.00mm, about 1.50mm and about 4.95mm, about 1.50mm and about 4.90mm, about 1.50mm and about 4.80 mm, about 1.50mm and about 4.70 mm, about 1.50mm and about 4.60mm, about 1.50mm and about 4.50 mm, about 1.50mm and about 4.40 mm, about 1.50mm and about 4.30 mm, about 1.50mm and about 4.20 mm, about 1.50mm and about 4.10 mm, about 1.50mm and about 4.00 mm, about 1.50mm and about 3.90 mm, about 1.50mm and about 3.80 mm, about 1.50mm and about 3.70 mm, about 1.50mm and about 3.60 mm, about 1.50mm and about 3.50 mm, about 1.50mm and about 3.40 mm, about 1.50mm and about 3.30 mm, about 1.50mm and about 3.20 mm, about 1.50mm and about 3.10 mm, about 1.50mm and about 3.00 mm, about 1.50mm and about 2.90 mm, about 1.50mm and about 2.80 mm, about 1.50mm and about 2.70 mm, about 1.50mm and about 2.60 mm, about 1.50mm and about 2.50 mm, about 1.50mm and about 2.40 mm, about 1.50mm and about 2.30 mm, about 1.50mm and about 2.20 mm, about 1.50mm and about 2.10 mm, about 1.50mm and about 2.00 mm, about 1.50mm and about 1.90 mm, about 1.50mm and about 1.80 mm, about 1.50mm and about 1.70 mm, or between about 1.50mm and about 1.60 mm.

The adhesive skin contact layer may comprise perforations each having a diameter of between about 1.60mm and about 5.00mm, about 1.60mm and about 4.95mm, about 1.60mm and about 4.90mm, about 1.60mm and about 4.80 mm, about 1.60mm and about 4.70 mm, about 1.60mm and about 4.60mm, about 1.60mm and about 4.50 mm, about 1.60mm and about 4.40 mm, about 1.60mm and about 4.30 mm, about 1.60mm and about 4.20 mm, about 1.60mm and about 4.10 mm, about 1.60mm and about 4.00 mm, about 1.60mm and about 3.90 mm, about 1.60mm and about 3.80 mm, about 1.60mm and about 3.70 mm, about 1.60mm and about 3.60 mm, about 1.60mm and about 3.50 mm, about 1.60mm and about 3.40 mm, about 1.60mm and about 3.30 mm, about 1.60mm and about 3.20 mm, about 1.60mm and about 3.10 mm, about 1.60mm and about 3.00 mm, about 1.60mm and about 2.90 mm, about 1.60mm and about 2.80 mm, about 1.60mm and about 2.70 mm, about 1.60mm and about 2.60 mm, about 1.60mm and about 2.50 mm, about 1.60mm and about 2.40 mm, about 1.60mm and about 2.30 mm, about 1.60mm and about 2.20 mm, about 1.60mm and about 2.10 mm, about 1.60mm and about 2.00 mm, about 1.60mm and about 1.90 mm, about 1.60mm and about 1.80 mm, or between about 1.60mm and about 1.70 mm.

The adhesive skin contact layer may comprise perforations each having a diameter of between about 1.70mm and about 5.00mm, about 1.70mm and about 4.95mm, about 1.70mm and about 4.90mm, about 1.70mm and about 4.80 mm, about 1.70mm and about 4.70 mm, about 1.70mm and about 4.60mm, about 1.70mm and about 4.50 mm, about 1.70mm and about 4.40 mm, about 1.70mm and about 4.30 mm, about 1.70mm and about 4.20 mm, about 1.70mm and about 4.10 mm, about 1.70mm and about 4.00 mm, about 1.70mm and about 3.90 mm, about 1.70mm and about 3.80 mm, about 1.70mm and about 3.70 mm, about 1.70mm and about 3.60 mm, about 1.70mm and about 3.50 mm, about 1.70mm and about 3.40 mm, about 1.70mm and about 3.30 mm, about 1.70mm and about 3.20 mm, about 1.70mm and about 3.10 mm, about 1.70mm and about 3.00 mm, about 1.70mm and about 2.90 mm, about 1.70mm and about 2.80 mm, about 1.70mm and about 2.70 mm, about 1.70mm and about 2.60 mm, about 1.70mm and about 2.50 mm, about 1.70mm and about 2.40 mm, about 1.70mm and about 2.30 mm, about 1.70mm and about 2.20 mm, about 1.70mm and about 2.10 mm, about 1.70mm and about 2.00 mm, about 1.70mm and about 1.90 mm, or between about 1.70mm and about 1.80 mm.

The adhesive skin contact layer may comprise perforations each having a diameter of between about 1.80mm and about 5.00mm, about 1.80mm and about 4.95mm, about 1.80mm and about 4.90mm, about 1.80mm and about 4.80 mm, about 1.80mm and about 4.70 mm, about 1.80mm and about 4.60mm, about 1.80mm and about 4.50 mm, about 1.80mm and about 4.40 mm, about 1.80mm and about 4.30 mm, about 1.80mm and about 4.20 mm, about 1.80mm and about 4.10 mm, about 1.80mm and about 4.00 mm, about 1.80mm and about 3.90 mm, about 1.80mm and about 3.80 mm, about 1.80mm and about 3.70 mm, about 1.80mm and about 3.60 mm, about 1.80mm and about 3.50 mm, about 1.80mm and about 3.40 mm, about 1.80mm and about 3.30 mm, about 1.80mm and about 3.20 mm, about 1.80mm and about 3.10 mm, about 1.80mm and about 3.00 mm, about 1.80mm and about 2.90 mm, about 1.80mm and about 2.80 mm, about 1.80mm and about 2.70 mm, about 1.80mm and about 2.60 mm, about 1.80mm and about 2.50 mm, about 1.80mm and about 2.40 mm, about 1.80mm and about 2.30 mm, about 1.80mm and about 2.20 mm, about 1.80mm and about 2.10 mm, about 1.80mm and about 2.00 mm, or between about 1.80mm and about 1.90 mm.

The adhesive skin contact layer may comprise perforations each having a diameter of between about 1.90mm and about 5.00mm, about 1.90mm and about 4.95mm, about 1.90mm and about 4.90mm, about 1.90mm and about 4.80 mm, about 1.90mm and about 4.70 mm, about 1.90mm and about 4.60mm, about 1.90mm and about 4.50 mm, about 1.90mm and about 4.40 mm, about 1.90mm and about 4.30 mm, about 1.90mm and about 4.20 mm, about 1.90mm and about 4.10 mm, about 1.90mm and about 4.00 mm, about 1.90mm and about 3.90 mm, about 1.90mm and about 3.80 mm, about 1.90mm and about 3.70 mm, about 1.90mm and about 3.60 mm, about 1.90mm and about 3.50 mm, about 1.90mm and about 3.40 mm, about 1.90mm and about 3.30 mm, about 1.90mm and about 3.20 mm, about 1.90mm and about 3.10 mm, about 1.90mm and about 3.00 mm, about 1.90mm and about 2.90 mm, about 1.90mm and about 2.80 mm, about 1.90mm and about 2.70 mm, about 1.90mm and about 2.60 mm, about 1.90mm and about 2.50 mm, about 1.90mm and about 2.40 mm, about 1.90mm and about 2.30 mm, about 1.90mm and about 2.20 mm, about 1.90mm and about 2.10 mm, or between about 1.90mm and about 2.00 mm.

The adhesive skin contact layer may comprise perforations each having a diameter of between about 2.00mm and about 5.00mm, about 2.00mm and about 4.95mm, about 2.00mm and about 4.90mm, about 2.00mm and about 4.80 mm, about 2.00mm and about 4.70 mm, about 2.00mm and about 4.60mm, about 2.00mm and about 4.50 mm, about 2.00mm and about 4.40 mm, about 2.00mm and about 4.30 mm, about 2.00mm and about 4.20 mm, about 2.00mm and about 4.10 mm, about 2.00mm and about 4.00 mm, about 2.00mm and about 3.90 mm, about 2.00mm and about 3.80 mm, about 2.00mm and about 3.70 mm, about 2.00mm and about 3.60 mm, about 2.00mm and about 3.50 mm, about 2.00mm and about 3.40 mm, about 2.00mm and about 3.30 mm, about 2.00mm and about 3.20 mm, about 2.00mm and about 3.10 mm, about 2.00mm and about 3.00 mm, about 2.00mm and about 2.90 mm, about 2.00mm and about 2.80 mm, about 2.00mm and about 2.70 mm, about 2.00mm and about 2.60 mm, about 2.00mm and about 2.50 mm, about 2.00mm and about 2.40 mm, about 2.00mm and about 2.30 mm, about 2.00mm and about 2.20 mm, or between about 2.00mm and about 2.10 mm.

The adhesive skin contact layer may comprise perforations each having a diameter of between about 2.10mm and about 5.00mm, about 2.10mm and about 4.95mm, about 2.10mm and about 4.90mm, about 2.10mm and about 4.80 mm, about 2.10mm and about 4.70 mm, about 2.10mm and about 4.60mm, about 2.10mm and about 4.50 mm, about 2.10mm and about 4.40 mm, about 2.10mm and about 4.30 mm, about 2.10mm and about 4.20 mm, about 2.10mm and about 4.10 mm, about 2.10mm and about 4.00 mm, about 2.10mm and about 3.90 mm, about 2.10mm and about 3.80 mm, about 2.10mm and about 3.70 mm, about 2.10mm and about 3.60 mm, about 2.10mm and about 3.50 mm, about 2.10mm and about 3.40 mm, about 2.10mm and about 3.30 mm, about 2.10mm and about 3.20 mm, about 2.10mm and about 3.10 mm, about 2.10mm and about 3.00 mm, about 2.10mm and about 2.90 mm, about 2.10mm and about 2.80 mm, about 2.10mm and about 2.70 mm, about 2.10mm and about 2.60 mm, about 2.10mm and about 2.50 mm, about 2.10mm and about 2.40 mm, about 2.10mm and about 2.30 mm, or between about 2.10mm and about 2.20 mm.

The adhesive skin contact layer may comprise perforations each having a diameter of between about 2.20mm and about 5.00mm, about 2.20mm and about 4.95mm, about 2.20mm and about 4.90mm, about 2.20mm and about 4.80 mm, about 2.20mm and about 4.70 mm, about 2.20mm and about 4.60mm, about 2.20mm and about 4.50 mm, about 2.20mm and about 4.40 mm, about 2.20mm and about 4.30 mm, about 2.20mm and about 4.20 mm, about 2.20mm and about 4.10 mm, about 2.20mm and about 4.00 mm, about 2.20mm and about 3.90 mm, about 2.20mm and about 3.80 mm, about 2.20mm and about 3.70 mm, about 2.20mm and about 3.60 mm, about 2.20mm and about 3.50 mm, about 2.20mm and about 3.40 mm, about 2.20mm and about 3.30 mm, about 2.20mm and about 3.20 mm, about 2.20mm and about 3.10 mm, about 2.20mm and about 3.00 mm, about 2.20mm and about 2.90 mm, about 2.20mm and about 2.80 mm, about 2.20mm and about 2.70 mm, about 2.20mm and about 2.60 mm, about 2.20mm and about 2.50 mm, about 2.20mm and about 2.40 mm, or between about 2.20mm and about 2.30 mm.

The adhesive skin contact layer may comprise perforations each having a diameter of between about 2.25mm and about 5.00mm, about 2.25mm and about 4.95mm, about 2.25mm and about 4.90mm, about 2.25mm and about 4.80 mm, about 2.25mm and about 4.70 mm, about 2.25mm and about 4.60mm, about 2.25mm and about 4.50 mm, about 2.25mm and about 4.40 mm, about 2.25mm and about 4.30 mm, about 2.25mm and about 4.20 mm, about 2.25mm and about 4.10 mm, about 2.25mm and about 4.00 mm, about 2.25mm and about 3.90 mm, about 2.25mm and about 3.80 mm, about 2.25mm and about 3.70 mm, about 2.25mm and about 3.60 mm, about 2.25mm and about 3.50 mm, about 2.25mm and about 3.40 mm, about 2.25mm and about 3.30 mm, about 2.25mm and about 3.20 mm, about 2.25mm and about 3.10 mm, about 2.25mm and about 3.00 mm, about 2.25mm and about 2.90 mm, about 2.25mm and about 2.80 mm, about 2.25mm and about 2.70 mm, about 2.25mm and about 2.60 mm, about 2.25mm and about 2.50 mm, about 2.25mm and about 2.40 mm, or between about 2.25mm and about 2.30 mm.

The adhesive skin contact layer may comprise perforations each having a diameter of between about 2.30mm and about 5.00mm, about 2.30mm and about 4.95mm, about 2.30mm and about 4.90mm, about 2.30mm and about 4.80 mm, about 2.30mm and about 4.70 mm, about 2.30mm and about 4.60mm, about 2.30mm and about 4.50 mm, about 2.30mm and about 4.40 mm, about 2.30mm and about 4.30 mm, about 2.30mm and about 4.20 mm, about 2.30mm and about 4.10 mm, about 2.30mm and about 4.00 mm, about 2.30mm and about 3.90 mm, about 2.30mm and about 3.80 mm, about 2.30mm and about 3.70 mm, about 2.30mm and about 3.60 mm, about 2.30mm and about 3.50 mm, about 2.30mm and about 3.40 mm, about 2.30mm and about 3.30 mm, about 2.30mm and about 3.20 mm, about 2.30mm and about 3.10 mm, about 2.30mm and about 3.00 mm, about 2.30mm and about 2.90 mm, about 2.30mm and about 2.80 mm, about 2.30mm and about 2.70 mm, about 2.30mm and about 2.60 mm, about 2.30mm and about 2.50 mm, or between about 2.30mm and about 2.40 mm.

The adhesive skin contact layer may comprise perforations each having a diameter of between about 2.40mm and about 5.00mm, about 2.40mm and about 4.95mm, about 2.40mm and about 4.90mm, about 2.40mm and about 4.80 mm, about 2.40mm and about 4.70 mm, about 2.40mm and about 4.60mm, about 2.40mm and about 4.50 mm, about 2.40mm and about 4.40 mm, about 2.40mm and about 4.30 mm, about 2.40mm and about 4.20 mm, about 2.40mm and about 4.10 mm, about 2.40mm and about 4.00 mm, about 2.40mm and about 3.90 mm, about 2.40mm and about 3.80 mm, about 2.40mm and about 3.70 mm, about 2.40mm and about 3.60 mm, about 2.40mm and about 3.50 mm, about 2.40mm and about 3.40 mm, about 2.40mm and about 3.30 mm, about 2.40mm and about 3.20 mm, about 2.40mm and about 3.10 mm, about 2.40mm and about 3.00 mm, about 2.40mm and about 2.90 mm, about 2.40mm and about 2.80 mm, about 2.40mm and about 2.70 mm, about 2.40mm and about 2.60 mm, or between about 2.40mm and about 2.50 mm.

The adhesive skin contact layer may comprise perforations each having a diameter of between about 2.50mm and about 5.00mm, about 2.50mm and about 4.95mm, about 2.50mm and about 4.90mm, about 2.50mm and about 4.80 mm, about 2.50mm and about 4.70 mm, about 2.50mm and about 4.60mm, about 2.50mm and about 4.50 mm, about 2.50mm and about 4.40 mm, about 2.50mm and about 4.30 mm, about 2.50mm and about 4.20 mm, about 2.50mm and about 4.10 mm, about 2.50mm and about 4.00 mm, about 2.50mm and about 3.90 mm, about 2.50mm and about 3.80 mm, about 2.50mm and about 3.70 mm, about 2.50mm and about 3.60 mm, about 2.50mm and about 3.50 mm, about 2.50mm and about 3.40 mm, about 2.50mm and about 3.30 mm, about 2.50mm and about 3.20 mm, about 2.50mm and about 3.10 mm, about 2.50mm and about 3.00 mm, about 2.50mm and about 2.90 mm, about 2.50mm and about 2.80 mm, about 2.50mm and about 2.70 mm, or between about 2.50mm and about 2.60 mm.

The adhesive skin contact layer may comprise perforations each having a diameter of between about 2.60mm and about 5.00mm, about 2.60mm and about 4.95mm, about 2.60mm and about 4.90mm, about 2.60mm and about 4.80 mm, about 2.60mm and about 4.70 mm, about 2.60mm and about 4.60mm, about 2.60mm and about 4.50 mm, about 2.60mm and about 4.40 mm, about 2.60mm and about 4.30 mm, about 2.60mm and about 4.20 mm, about 2.60mm and about 4.10 mm, about 2.60mm and about 4.00 mm, about 2.60mm and about 3.90 mm, about 2.60mm and about 3.80 mm, about 2.60mm and about 3.70 mm, about 2.60mm and about 3.60 mm, about 2.60mm and about 3.50 mm, about 2.60mm and about 3.40 mm, about 2.60mm and about 3.30 mm, about 2.60mm and about 3.20 mm, about 2.60mm and about 3.10 mm, about 2.60mm and about 3.00 mm, about 2.60mm and about 2.90 mm, about 2.60mm and about 2.80 mm, or between about 2.60mm and about 2.70 mm.

The adhesive skin contact layer may comprise perforations each having a diameter of between about 2.70mm and about 5.00mm, about 2.70mm and about 4.95mm, about 2.70mm and about 4.90mm, about 2.70mm and about 4.80 mm, about 2.70mm and about 4.70 mm, about 2.70mm and about 4.60mm, about 2.70mm and about 4.50 mm, about 2.70mm and about 4.40 mm, about 2.70mm and about 4.30 mm, about 2.70mm and about 4.20 mm, about 2.70mm and about 4.10 mm, about 2.70mm and about 4.00 mm, about 2.70mm and about 3.90 mm, about 2.70mm and about 3.80 mm, about 2.70mm and about 3.70 mm, about 2.70mm and about 3.60 mm, about 2.70mm and about 3.50 mm, about 2.70mm and about 3.40 mm, about 2.70mm and about 3.30 mm, about 2.70mm and about 3.20 mm, about 2.70mm and about 3.10 mm, about 2.70mm and about 3.00 mm, about 2.70mm and about 2.90 mm, or between about 2.70mm and about 2.80 mm.

The adhesive skin contact layer may comprise perforations each having a diameter of between about 2.80mm and about 5.00mm, about 2.80mm and about 4.95mm, about 2.80mm and about 4.90mm, about 2.80mm and about 4.80 mm, about 2.80mm and about 4.70 mm, about 2.80mm and about 4.60mm, about 2.80mm and about 4.50 mm, about 2.80mm and about 4.40 mm, about 2.80mm and about 4.30 mm, about 2.80mm and about 4.20 mm, about 2.80mm and about 4.10 mm, about 2.80mm and about 4.00 mm, about 2.80mm and about 3.90 mm, about 2.80mm and about 3.80 mm, about 2.80mm and about 3.70 mm, about 2.80mm and about 3.60 mm, about 2.80mm and about 3.50 mm, about 2.80mm and about 3.40 mm, about 2.80mm and about 3.30 mm, about 2.80mm and about 3.20 mm, about 2.80mm and about 3.10 mm, about 2.80mm and about 3.00 mm, or between about 2.80mm and about 2.90 mm.

The adhesive skin contact layer may comprise perforations each having a diameter of between about 2.90mm and about 5.00mm, about 2.90mm and about 4.95mm, about 2.90mm and about 4.90mm, about 2.90mm and about 4.80 mm, about 2.90mm and about 4.70 mm, about 2.90mm and about 4.60mm, about 2.90mm and about 4.50 mm, about 2.90mm and about 4.40 mm, about 2.90mm and about 4.30 mm, about 2.90mm and about 4.20 mm, about 2.90mm and about 4.10 mm, about 2.90mm and about 4.00 mm, about 2.90mm and about 3.90 mm, about 2.90mm and about 3.80 mm, about 2.90mm and about 3.70 mm, about 2.90mm and about 3.60 mm, about 2.90mm and about 3.50 mm, about 2.90mm and about 3.40 mm, about 2.90mm and about 3.30 mm, about 2.90mm and about 3.20 mm, about 2.90mm and about 3.10 mm, or between about 2.90mm and about 3.00 mm.

The adhesive skin contact layer may comprise perforations each having a diameter of between about 3.00mm and about 5.00mm, about 3.00mm and about 4.95mm, about 3.00mm and about 4.90mm, about 3.00mm and about 4.80 mm, about 3.00mm and about 4.70 mm, about 3.00mm and about 4.60mm, about 3.00mm and about 4.50 mm, about 3.00mm and about 4.40 mm, about 3.00mm and about 4.30 mm, about 3.00mm and about 4.20 mm, about 3.00mm and about 4.10 mm, about 3.00mm and about 4.00 mm, about 3.00mm and about 3.90 mm, about 3.00mm and about 3.80 mm, about 3.00mm and about 3.70 mm, about 3.00mm and about 3.60 mm, about 3.00mm and about 3.50 mm, about 3.00mm and about 3.40 mm, about 3.00mm and about 3.30 mm, about 3.00mm and about 3.20 mm, or between about 3.00mm and about 3.10 mm.

The adhesive skin contact layer may comprise perforations each having a diameter of between about 3.10mm and about 5.00mm, about 3.10mm and about 4.95mm, about 3.10mm and about 4.90mm, about 3.10mm and about 4.80 mm, about 3.10mm and about 4.70 mm, about 3.10mm and about 4.60mm, about 3.10mm and about 4.50 mm, about 3.10mm and about 4.40 mm, about 3.10mm and about 4.30 mm, about 3.10mm and about 4.20 mm, about 3.10mm and about 4.10 mm, about 3.10mm and about 4.00 mm, about 3.10mm and about 3.90 mm, about 3.10mm and about 3.80 mm, about 3.10mm and about 3.70 mm, about 3.10mm and about 3.60 mm, about 3.10mm and about 3.50 mm, about 3.10mm and about 3.40 mm, about 3.10mm and about 3.30 mm, or between about 3.10mm and about 3.20 mm.

The adhesive skin contact layer may comprise perforations each having a diameter of between about 3.20mm and about 5.00mm, about 3.20mm and about 4.95mm, about 3.20mm and about 4.90mm, about 3.20mm and about 4.80 mm, about 3.20mm and about 4.70 mm, about 3.20mm and about 4.60mm, about 3.20mm and about 4.50 mm, about 3.20mm and about 4.40 mm, about 3.20mm and about 4.30 mm, about 3.20mm and about 4.20 mm, about 3.20mm and about 4.10 mm, about 3.20mm and about 4.00 mm, about 3.20mm and about 3.90 mm, about 3.20mm and about 3.80 mm, about 3.20mm and about 3.70 mm, about 3.20mm and about 3.60 mm, about 3.20mm and about 3.50 mm, about 3.20mm and about 3.40 mm, or between about 3.20mm and about 3.30 mm.

The adhesive skin contact layer may comprise perforations each having a diameter of between about 3.30mm and about 5.00mm, about 3.30mm and about 4.95mm, about 3.30mm and about 4.90mm, about 3.30mm and about 4.80 mm, about 3.30mm and about 4.70 mm, about 3.30mm and about 4.60mm, about 3.30mm and about 4.50 mm, about 3.30mm and about 4.40 mm, about 3.30mm and about 4.30 mm, about 3.30mm and about 4.20 mm, about 3.30mm and about 4.10 mm, about 3.30mm and about 4.00 mm, about 3.30mm and about 3.90 mm, about 3.30mm and about 3.80 mm, about 3.30mm and about 3.70 mm, about 3.30mm and about 3.60 mm, about 3.30mm and about 3.50 mm, or between about 3.30mm and about 3.40 mm.

The adhesive skin contact layer may comprise perforations each having a diameter of between about 3.40mm and about 5.00mm, about 3.40mm and about 4.95mm, about 3.40mm and about 4.90mm, about 3.40mm and about 4.80 mm, about 3.40mm and about 4.70 mm, about 3.40mm and about 4.60mm, about 3.40mm and about 4.50 mm, about 3.40mm and about 4.40 mm, about 3.40mm and about 4.30 mm, about 3.40mm and about 4.20 mm, about 3.40mm and about 4.10 mm, about 3.40mm and about 4.00 mm, about 3.40mm and about 3.90 mm, about 3.40mm and about 3.80 mm, about 3.40mm and about 3.70 mm, about 3.40mm and about 3.60 mm, or between about 3.40mm and about 3.50 mm.

The adhesive skin contact layer may comprise perforations each having a diameter of between about 3.50mm and about 5.00mm, about 3.50mm and about 4.95mm, about 3.50mm and about 4.90mm, about 3.50mm and about 4.80 mm, about 3.50mm and about 4.70 mm, about 3.50mm and about 4.60mm, about 3.50mm and about 4.50 mm, about 3.50mm and about 4.40 mm, about 3.50mm and about 4.30 mm, about 3.50mm and about 4.20 mm, about 3.50mm and about 4.10 mm, about 3.50mm and about 4.00 mm, about 3.50mm and about 3.90 mm, about 3.50mm and about 3.80 mm, about 3.50mm and about 3.70 mm, or between about 3.50mm and about 3.60 mm.

The adhesive skin contact layer may comprise perforations each having a diameter of between about 3.60mm and about 5.00mm, about 3.60mm and about 4.95mm, about 3.60mm and about 4.90mm, about 3.60mm and about 4.80 mm, about 3.60mm and about 4.70 mm, about 3.60mm and about 4.60mm, about 3.60mm and about 4.50 mm, about 3.60mm and about 4.40 mm, about 3.60mm and about 4.30 mm, about 3.60mm and about 4.20 mm, about 3.60mm and about 4.10 mm, about 3.60mm and about 4.00 mm, about 3.60mm and about 3.90 mm, about 3.60mm and about 3.80 mm, or between about 3.60mm and about 3.70 mm.

The adhesive skin contact layer may comprise perforations each having a diameter of between about 3.70mm and about 5.00mm, about 3.70mm and about 4.95mm, about 3.70mm and about 4.90mm, about 3.70mm and about 4.80 mm, about 3.70mm and about 4.70 mm, about 3.70mm and about 4.60mm, about 3.70mm and about 4.50 mm, about 3.70mm and about 4.40 mm, about 3.70mm and about 4.30 mm, about 3.70mm and about 4.20 mm, about 3.70mm and about 4.10 mm, about 3.70mm and about 4.00 mm, about 3.70mm and about 3.90 mm, or between about 3.70mm and about 3.80 mm.

The adhesive skin contact layer may comprise perforations each having a diameter of between about 3.80mm and about 5.00mm, about 3.80mm and about 4.95mm, about 3.80mm and about 4.90mm, about 3.80mm and about 4.80 mm, about 3.80mm and about 4.70 mm, about 3.80mm and about 4.60mm, about 3.80mm and about 4.50 mm, about 3.80mm and about 4.40 mm, about 3.80mm and about 4.30 mm, about 3.80mm and about 4.20 mm, about 3.80mm and about 4.10 mm, about 3.80mm and about 4.00 mm, or between about 3.80mm and about 3.90 mm.

The adhesive skin contact layer may comprise perforations each having a diameter of between about 3.90mm and about 5.00mm, about 3.90mm and about 4.95mm, about 3.90mm and about 4.90mm, about 3.90mm and about 4.80 mm, about 3.90mm and about 4.70 mm, about 3.90mm and about 4.60mm, about 3.90mm and about 4.50 mm, about 3.90mm and about 4.40 mm, about 3.90mm and about 4.30 mm, about 3.90mm and about 4.20 mm, about 3.90mm and about 4.10 mm, or between about 3.90mm and about 4.00 mm.

The adhesive skin contact layer may comprise perforations each having a diameter of between about 4.00mm and about 5.00mm, about 4.00mm and about 4.95mm, about 4.00mm and about 4.90mm, about 4.00mm and about 4.80 mm, about 4.00mm and about 4.70 mm, about 4.00mm and about 4.60mm, about 4.00mm and about 4.50 mm, about 4.00mm and about 4.40 mm, about 4.00mm and about 4.30 mm, about 4.00mm and about 4.20 mm, or between about 4.00mm and about 4.10 mm.

The adhesive skin contact layer may comprise perforations each having a diameter of between about 4.10mm and about 5.00mm, about 4.10mm and about 4.95mm, about 4.10mm and about 4.90mm, about 4.10mm and about 4.80 mm, about 4.10mm and about 4.70 mm, about 4.10mm and about 4.60mm, about 4.10mm and about 4.50 mm, about 4.10mm and about 4.40 mm, about 4.10mm and about 4.30 mm, or between about 4.10mm and about 4.20 mm.

The adhesive skin contact layer may comprise perforations each having a diameter of between about 4.20mm and about 5.00mm, about 4.20mm and about 4.95mm, about 4.20mm and about 4.90mm, about 4.20mm and about 4.80 mm, about 4.20mm and about 4.70 mm, about 4.20mm and about 4.60mm, about 4.20mm and about 4.50 mm, about 4.20mm and about 4.40 mm, or between about 4.20mm and about 4.30 mm.

The adhesive skin contact layer may comprise perforations each having a diameter of between about 4.30mm and about 5.00mm, about 4.30mm and about 4.95mm, about 4.30mm and about 4.90mm, about 4.30mm and about 4.80 mm, about 4.30mm and about 4.70 mm, about 4.30mm and about 4.60mm, about 4.30mm and about 4.50 mm, or between about 4.30mm and about 4.40 mm.

The adhesive skin contact layer may comprise perforations each having a diameter of between about 4.40mm and about 5.00mm, about 4.40mm and about 4.95mm, about 4.40mm and about 4.90mm, about 4.40mm and about 4.80 mm, about 4.40mm and about 4.70 mm, about 4.40mm and about 4.60mm, or between about 4.40mm and about 4.50 mm.

The adhesive skin contact layer may comprise perforations each having a diameter of between about 4.50mm and about 5.00mm, about 4.50mm and about 4.95mm, about 4.50mm and about 4.90mm, about 4.50mm and about 4.80 mm, about 4.50mm and about 4.70 mm, or between about 4.50mm and about 4.60mm.

The adhesive skin contact layer may comprise perforations each having a diameter of between about 4.60mm and about 5.00mm, about 4.60mm and about 4.95mm, about 4.60mm and about 4.90mm, about 4.60mm and about 4.80 mm, or between about 4.60mm and about 4.70 mm.

The adhesive skin contact layer may comprise perforations each having a diameter of between about 4.70mm and about 5.00mm, about 4.70mm and about 4.95mm, about 4.70mm and about 4.90mm, or between about 4.70mm and about 4.80 mm.

The adhesive skin contact layer may comprise perforations each having a diameter of between about 4.80mm and about 5.00mm, about 4.80mm and about 4.95mm, or between about 4.80mm and about 4.90mm.

The adhesive skin contact layer may comprise perforations each having a diameter of between about 4.90mm and about 5.00mm, or between about 4.90mm and about 4.95mm.

The adhesive skin contact layer may comprise perforations each having a diameter of between about 4.95mm and about 5.00mm.

The perforations may be arranged in a square-packed layout or a triangle packed layout.

A square-packed layout means that all, or a significant proportion, for example at least about 60%, about 65%, about 70%, about 75%, about 80%, about 85%, about 90%, or at least about 95%, of the perforations are arranged in parallel rows and columns.

A triangle-packed layout means that all, or a significant proportion, for example at least about 60%, about 65%, about 70%, about 75%, about 80%, about 85%, about 90%, or at least about 95%, of the perforations are arranged in adjacent rows of perforations which are offset from one another. By this we mean that the perforations in a first row are not arranged adjacent to the perforations in an adjacent second row but instead the perforations in the first row are arranged adjacent to the gap between perforations in the second row or adjacent to part of the gap and part of a perforation in the second row.

Each perforation may comprise an opening comprising a 2D shape selected from the group comprising: a circle, an oval, a triangle, a square, a rectangle, a hexagon or an octagon.

The person skilled in the art will understand that the size of the opening may be defined depending on the shape of the opening. In some embodiments, the size of a circle is the diameter. In other embodiments, the size of an oval is the longer diameter. In further embodiments, the size of a hexagon is the longest diagonal; and the size of a triangle, a square, a rectangle, an octagon or any other polygon perforation is the longest side.

The perforations may have a pitch (i.e., the distance between the centre point of a perforation and the centre point of an adjacent perforation) in a first direction of about 6.0mm, about 6.5mm, about 7.0mm, about 7.5mm, about 8.0mm, about 8.5mm, about 9.0mm, about 9.5mm, about 10.0mm, about 10.5mm, about 11.0mm, about 11.5mm, or about 12.0mm.

The perforations may have a pitch in a first direction of at least about 6.0mm, about 6.5mm, about 7.0mm, about 7.5mm, about 8.0mm, about 8.5mm, about 9.0mm, about 9.5mm, about 10.0mm, about 10.5mm, about 11.0mm, about 11.5mm, or at least about 12.0mm.

The perforations may have a pitch in a first direction of no more than about 6.0mm, about 6.5mm, about 7.0mm, about 7.5mm, about 8.0mm, about 8.5mm, about 9.0mm, about 9.5mm, about 10.0mm, about 10.5mm, about 11.0mm, about 11.5mm, or no more than about 12.0mm.

The perforations may have a pitch in a first direction of between about 6.0mm and about 10.0mm, about 6.5mm and about 9.5mm, about 7.0mm and about 9.0mm, about 7.5mm and about 8.5mm, about 7.75mm and about 8.25mm, or about 8.0mm.

The perforations may have a pitch in a first direction of between about 6.0mm and about 10.0mm, about 6.0mm to about 9.5mm, about 6.0mm to about 9.0mm, about 6.0mm to about 8.50mm, about 6.0mm to about 8.0mm, about 6.0mm to about 7.5mm, about 6.0mm to about 7.0mm, or between about 6.0mm to about 6.5mm.

The perforations may have a pitch in a first direction of between about 6.5mm and about 10.0mm, about 6.5mm to about 9.5mm, about 6.5mm to about 9.0mm, about 6.5mm to about 8.50mm, about 6.5mm to about 8.0mm, about 6.5mm to about 7.5mm, or between about 6.5mm to about 7.0mm.

The perforations may have a pitch in a first direction of between about 7.0mm and about 10.0mm, about 7.0mm to about 9.5mm, about 7.0mm to about 9.0mm, about 7.0mm to about 8.50mm, about 7.0mm to about 8.0mm, or between about 7.0mm to about 7.5mm.

The perforations may have a pitch in a first direction of between about 7.5mm and about 10.0mm, about 7.5mm to about 9.5mm, about 7.5mm to about 9.0mm, about 7.5mm to about 8.50mm, or between about 7.5mm to about 8.0mm.

The perforations may have a pitch in a first direction of between about 8.0mm and about 10.0mm, about 8.0mm to about 9.5mm, about 8.0mm to about 9.0mm, or between about 8.0mm to about 8.50mm.

The perforations may have a pitch in a first direction of between about 8.5mm and about 10.0mm, about 8.5mm to about 9.5mm, or between about 8.5mm to about 9.0mm.

The perforations may have a pitch in a first direction of between about 9.0mm and about 10.0mm, or between about 9.0mm to about 9.5mm.

The perforations may have a pitch in a first direction of between about 9.5mm and about 10.0mm.

The perforations may have a pitch in a second direction of about 3.0mm, about 3.5mm, about 4.0mm, about 4.5mm, about 5.0mm, about 5.5mm, about 5.6mm, about 6.0mm, about 6.5mm, about 7.0mm, about 7.5mm, about 8.0mm, about 8.5mm, about 9.0mm, about 9.5mm, or about 10.0mm.

The perforations may have a pitch in a second direction of at least about 3.0mm, about 3.5mm, about 4.0mm, about 4.5mm, about 5.0mm, about 5.5mm, about 5.6mm, about 6.0mm, about 6.5mm, about 7.0mm, about 7.5mm, about 8.0mm, about 8.5mm, about 9.0mm, about 9.5mm, or at least about 10.0mm.

The perforations may have a pitch in a second direction of no more than about 3.0mm, about 3.5mm, about 4.0mm, about 4.5mm, about 5.0mm, about 5.5mm, about 5.6mm, about 6.0mm, about 6.5mm, about 7.0mm, about 7.5mm, about 8.0mm, about 8.5mm, about 9.0mm, about 9.5mm, or no more than about 10.0mm.

The perforations may have a pitch in a second direction of between about 4.00mm and about 7.00mm, about 4.25mm and about 6.75mm, about 4.50mm and about 6.50mm, about 4.75mm and about 6.25mm, about 5.00mm and about 6.00mm, about 5.25mm and about 5.75mm, about 5.40mm and about 5.70mm, about 5.50mm and about 5.65mm, about 5.55mm and about 5.62mm, or about 5.60mm.

The perforations may have a pitch in a second direction of between about 3.0mm and about 10.0mm, about 3.0mm and about 9.5mm, about 3.0mm and about 9.0mm, about 3.0mm and about 8.5 mm, about 3.0mm and about 8.0 mm, about 3.0mm and about 7.5mm, about 3.0mm and about 7.0mm, about 3.0mm and about 6.5mm, about 3.0mm and about 6.0mm, about 3.0mm and about 5.5mm, about 3.0mm and about 5.0mm, about 3.0mm and about 4.5mm, about 3.0mm and about 4.0mm, or between about 3.0mm and about 3.5mm.

The perforations may have a pitch in a second direction of between about 3.5mm and about 10.0mm, about 3.5mm and about 9.5mm, about 3.5mm and about 9.0mm, about 3.5mm and about 8.5 mm, about 3.5mm and about 8.0 mm, about 3.5mm and about 7.5mm, about 3.5mm and about 7.0mm, about 3.5mm and about 6.5mm, about 3.5mm and about 6.0mm, about 3.5mm and about 5.5mm, about 3.5mm and about 5.0mm, about 3.5mm and about 4.5mm, or between about 3.5mm and about 4.0mm.

The perforations may have a pitch in a second direction of between about 4.0mm and about 10.0mm, about 4.0mm and about 9.5mm, about 4.0mm and about 9.0mm, about 4.0mm and about 8.5 mm, about 4.0mm and about 8.0 mm, about 4.0mm and about 7.5mm, about 4.0mm and about 7.0mm, about 4.0mm and about 6.5mm, about 4.0mm and about 6.0mm, about 4.0mm and about 5.5mm, about 4.0mm and about 5.0mm, or between about 4.5mm and about 4.5mm.

The perforations may have a pitch in a second direction of between about 4.5mm and about 10.0mm, about 4.5mm and about 9.5mm, about 4.5mm and about 9.0mm, about 4.5mm and about 8.5 mm, about 4.5mm and about 8.0 mm, about 4.5mm and about 7.5mm, about 4.5mm and about 7.0mm, about 4.5mm and about 6.5mm, about 4.5mm and about 6.0mm, about 4.5mm and about 5.5mm, or between about 4.5mm and about 5.0mm.

The perforations may have a pitch in a second direction of between about 5.0mm and about 10.0mm, about 5.0mm and about 9.5mm, about 5.0mm and about 9.0mm, about 5.0mm and about 8.5 mm, about 5.0mm and about 8.0 mm, about 5.0mm and about 7.5mm, about 5.0mm and about 7.0mm, about 5.0mm and about 6.5mm, about 5.0mm and about 6.0mm, or between about 5.5mm and about 5.5mm.

The perforations may have a pitch in a second direction of between about 5.5mm and about 10.0mm, about 5.5mm and about 9.5mm, about 5.5mm and about 9.0mm, about 5.5mm and about 8.5 mm, about 5.5mm and about 8.0 mm, about 5.5mm and about 7.5mm, about 5.5mm and about 7.0mm, about 5.5mm and about 6.5mm, or between about 5.5mm and about 6.0mm.

The perforations may have a pitch in a second direction of between about 6.0mm and about 10.0mm, about 6.0mm and about 9.5mm, about 6.0mm and about 9.0mm, about 6.0mm and about 8.5 mm, about 6.0mm and about 8.0 mm, about 6.0mm and about 7.5mm, about 6.0mm and about 7.0mm, or between about 6.0mm and about 6.5mm.

The perforations may have a pitch in a second direction of between about 6.5mm and about 10.0mm, about 6.5mm and about 9.5mm, about 6.5mm and about 9.0mm, about 6.5mm and about 8.5 mm, about 6.5mm and about 8.0 mm, about 6.5mm and about 7.5mm, or between about 6.5mm and about 7.0mm.

The perforations may have a pitch in a second direction of between about 7.0mm and about 10.0mm, about 7.0mm and about 9.5mm, about 7.0mm and about 9.0mm, about 7.0mm and about 8.5 mm, about 7.0mm and about 8.0 mm, or between about 7.0mm and about 7.5mm.

The perforations may have a pitch in a second direction of between about 7.5mm and about 10.0mm, about 7.5mm and about 9.5mm, about 7.5mm and about 9.0mm, about 7.5mm and about 8.5 mm, or between about 7.5mm and about 8.0 mm.

The perforations may have a pitch in a second direction of between about 8.0mm and about 10.0mm, about 8.0mm and about 9.5mm, about 8.0mm and about 9.0mm, or between about 8.0mm and about 8.5 mm.

The perforations may have a pitch in a second direction of between about 8.5mm and about 10.0mm, about 8.5mm and about 9.5mm, or between about 8.5mm and about 9.0mm.

The perforations may have a pitch in a second direction of between about 9.0mm and about 10.0mm, or between about 9.0mm and about 9.5mm.

The perforations may have a pitch in a second direction of between about 9.5mm and about 10.0mm.

Advantageously, the pitch of the perforations of the present invention, in particular in embodiments where the pitch is between about 7mm and 9mm, in particular about 8mm, in a first direction, and between about 5.00mm and about 6.00mm, in particular about 5.6mm, in a second direction, provides a good ratio of perforation : no-perforation of the adhesive skin contact layer. Beneficially, this provides for efficient moisture evaporation (for example to prevent pooling) and strong adhesion to the patient. Surprisingly, and advantageously, it has been found that the adhesive skin contact layer of the present invention, in particular in embodiments having the above-described pitch of the perforations, remains adhered to a user for at least seven days.

The adhesive skin contact layer may comprise at least two perforations having different diameters.

The difference in the diameter of the largest perforation and the smallest perforation may be no more than about 4.50mm, about 4.40mm, about mm, about 4.35 mm, about 4.30 mm, about 4.25 mm, about 4.20 mm, about 4.15 mm, about 4.10 mm, about 4.05 mm, about 4.00 mm, about 3.95 mm, about 3.90 mm, about 3.85 mm, about 3.80 mm, about 3.75 mm, about 3.70 mm, about 3.65 mm, about 3.60 mm, about 3.55 mm, about 3.50 mm, about 3.45 mm, about 3.40 mm, about 3.35 mm, about 3.30 mm, about 3.25 mm, about 3.20 mm, about 3.15 mm, about 3.10 mm, about 3.05 mm, about 3.00 mm, about 2.95 mm, about 2.90 mm, about 2.85 mm, about 2.80 mm, about 2.75 mm, about 2.70 mm, about 2.65 mm, about 2.60 mm, about 2.55 mm, about 2.50 mm, about 2.45 mm, about 2.40 mm, about 2.35 mm, about 2.30 mm, about 2.25 mm, about 2.20 mm, about 2.15 mm, about 2.10 mm, about 2.05 mm, about 2.00 mm, about 1.95 mm, about 1.90 mm, about 1.85 mm, about 1.80 mm, about 1.75 mm, about 1.70 mm, about 1.65 mm, about 1.60 mm, about 1.55 mm, about 1.50 mm, about 1.45 mm, about 1.40 mm, about 1.35 mm, about 1.30 mm, about 1.25 mm, about 1.20 mm, about 1.15 mm, about 1.10 mm, about 1.05 mm, about 1.00 mm, about 0.95 mm, about 0.90 mm, about 0.85 mm, about 0.80 mm, about 0.75 mm, about 0.70 mm, about 0.65 mm, about 0.60 mm, about 0.55 mm, about 0.50 mm, about 0.45 mm, about 0.40 mm, about 0.35 mm, about 0.30 mm, about 0.25 mm, about 0.20 mm, about 0.15 mm, about 0.10 mm, or no more than about 0.05 mm.

The perforations may have a total area of about 1300 mm², about 1350 mm², about 1400 mm², about 1450 mm², about 1500 mm², about 1550 mm², about 1600 mm², about 1650 mm², about 1700 mm², about 1750 mm², about 1800 mm², about 1850 mm², about 1900 mm², about 1950 mm², about 2000 mm², about 2050 mm², about 2100 mm², about 2150 mm², about 2200 mm², about 2250 mm², about 2300 mm², about 2350 mm², about 2400 mm², about 2450 mm², or about 2500 mm².

The perforations may have a total area of between about 1300 mm² and about 2500 mm², about 1400 mm² and about 2450 mm², about 1500 mm² and about 2400 mm², about 1600 mm² and about 2350 mm², about 1700 mm² and about 2300 mm², about 1800 mm² and about 2250 mm², about 1900 mm² and about 2200 mm², about 2000 mm² and about 2150 mm², or about 2100 mm².

The degree of perforation may be between about 5% and about 50%, about 6% and about 45%, about 7% and about 40%, about 8% and about 35%, about 9% and about 30%, about 10% and about 25%, about 10% and about 20%, about 10% and about 15%, or about 12%, of the area of the adhesive skin contact layer.

The degree of perforation may be about 5%, about 6%, about 7%, about 8%, about 9%, about 10%, about 12%, about 14%, about 16%, about 18%, about 20%, about 22%, about 24%, about 26%, about 28%, about 30%, about 32%, about 34%, about 36%, about 38%, about 40%, about 42%, about 44%, about 46%, about 48%, or about 50%, of the area of the adhesive skin contact layer.

The degree of perforation may be at least about 5%, about 6%, about 7%, about 8%, about 9%, about 10%, about 12%, about 14%, about 16%, about 18%, about 20%, about 22%, about 24%, about 26%, about 28%, about 30%, about 32%, about 34%, about 36%, about 38%, about 40%, about 42%, about 44%, about 46%, about 48%, or at least about 50%, of the area of the adhesive skin contact layer.

The degree of perforation may be no more than about 5%, about 6%, about 7%, about 8%, about 9%, about 10%, about 12%, about 14%, about 16%, about 18%, about 20%, about 22%, about 24%, about 26%, about 28%, about 30%, about 32%, about 34%, about 36%, about 38%, about 40%, about 42%, about 44%, about 46%, about 48%, or no more than about 50%, of the area of the adhesive skin contact layer.

The adhesive skin contact layer may have a thickness of about 0.5mm, about 0.6mm, about 0.7mm, about 0.8mm, about 0.9mm, about 1.0mm, about 1.1mm, about 1.2mm, about 1.3mm, about 1.4mm, or about 1.5mm.

The adhesive skin contact layer may have a thickness of at least about 0.5mm, about 0.6mm, about 0.7mm, about 0.8mm, about 0.9mm, about 1.0mm, about 1.1mm, about 1.2mm, about 1.3mm, about 1.4mm, or at least about 1.5mm.

The adhesive skin contact layer may have a thickness of no more than about 0.5mm, about 0.6mm, about 0.7mm, about 0.8mm, about 0.9mm, about 1.0mm, about 1.1mm, about 1.2mm, about 1.3mm, about 1.4mm, or no more than about 1.5mm.

The adhesive skin contact layer may have a thickness of between about 0.5mm and about 1.5mm, about 0.6mm and about 1.4mm, about 0.7mm and about 1.3mm, about 0.8mm and about 1.2mm, about 0.9mm and about 1.1mm, or about 1.0mm.

The perforations may be formed by punch perforating, a hot pin process, a laser ablation process or an ultrasound process. In particular, the perforations may be formed by mechanical punch perforating. Advantageously, mechanical punch perforating prevents, or at least significantly reduces, formation of a doughnut or volcano structure around the perforations after their formation. The perforations may be formed by mechanical punch perforating an adhesive layer comprising a release liner covering at least one surface of the adhesive layer. Following mechanical punch perforating the adhesive layer, the release liner may be removed from the at least one surface of the adhesive layer to remove any 'slugs' that remain after mechanical punch perforating.

The perforations may be formed by punch perforating, a hot pin process, or an ultrasound process.

Each of the perforations may be the same size, or at least substantially the same size.

The adhesive skin contact layer may be any shape, such as a circle, an oval, a triangle, a square, a rectangle, a hexagon, an octagon or any irregular shape, for example an irregular quadrilateral.

The adhesive skin contact layer may comprise a window. Thus, the adhesive skin contact layer may be an adhesive skin contact border layer having a perimetral shape. The window may be located central relative to the periphery of the adhesive skin contact layer. The border layer may comprise an outer periphery which defines the outer shape of the border, for example square, rectangle, circle. The border layer may comprise an inner periphery which defines the shape of the window. The window may be of the same, or similar shape, to that of the outer periphery of the border layer. In use, the wound dressing may be adhered to the dermal surface such that a wound site is located within the window. As such, the adhesive skin contact layer is not in direct contact with the wound site, but does partially, or wholly, surround it. Thus, the adhesive skin contact layer may be in contact with intact peri-wound skin only. Advantageously, this means that the wound dressing does not directly adhere to the wound site, therefore, removal of the wound dressing from a patient does not damage the wound and minimises discomfort to the patient. The window allows the adhesive skin contact layer to be adhered around a wound, such that the wound site is placed within the window, therefore, allowing the patient's wound to be in contact, or be in fluid communication with, for example and preferably, the wound contact layer when the wound dressing is applied to the skin of a patient.

In embodiments comprising a rectangle window, there may be provided a greater overlap of the absorbent structure (i.e., the structure which may comprise a wound contact later, transmission layer and/or superabsorbent layer) and the adhesive skin contact layer. The increased overlap may be found at or toward corresponding corner portions of the adhesive skin contact layer and the absorbent structure. Advantageously, the greater overlap allows for a greater surface area for adhesion between the adhesive skin contact layer and the absorbent structure, therefore, providing greater structural integrity to the wound dressing. The greater structural integrity means that the absorbent structure maintains its position within the wound dressing with reduced possibility of the absorbent structure becoming dislodged from its position within the wound dressing when in use, in particular when in use when the wound dressing is wet with exudate or other fluids.

The window may have an area of between about 900 mm² and about 50,000 mm², about 1600 mm² and about 45,000 mm², about 2500 mm² and about 40,000 mm², about 3600 mm² and about 35,000 mm², about 4900 mm² and about 30,000 mm², about 6400 mm² and about 25,000 mm², about 8100 mm² and about 20,000 mm², about 10,000 mm² and about 15,000 mm², or about 12,500 mm².

The window may have an area of about 900 mm², about 1200 mm², about 1600 mm², about 2000 mm², about 2500 mm², about 3000 mm², about 3600 mm², about 4000 mm², about 4400 mm², about 4900 mm², about 5400 mm², about 5900 mm², about 6400 mm², about 6900 mm², about 7500 mm², about 8100 mm², about 8700 mm², about 9300 mm², about 10,000 mm², about 12,500 mm², about 15,000 mm², about 17,500 mm², about 20,000 mm², about 22,500 mm², about 25,000 mm², about 27,500 mm², about 30,000 mm², about 32,500 mm², about 35,000 mm², about 37,500 mm², about 40,000 mm², about 42,500 mm², about 45,000 mm², about 47,500 mm², or about 50,000 mm².

The window may have an area of at least about 900 mm², about 1200 mm², about 1600 mm², about 2000 mm², about 2500 mm², about 3000 mm², about 3600 mm², about 4000 mm², about 4400 mm², about 4900 mm², about 5400 mm², about 5900 mm², about 6400 mm², about 6900 mm², about 7500 mm², about 8100 mm², about 8700 mm², about 9300 mm², about 10,000 mm², about 12,500 mm², about 15,000 mm², about 17,500 mm², about 20,000 mm², about 22,500 mm², about 25,000 mm², about 27,500 mm², about 30,000 mm², about 32,500 mm², about 35,000 mm², about 37,500 mm², about 40,000 mm², about 42,500 mm², about 45,000 mm², about 47,500 mm², or at least about 50,000 mm².

The window may have an area of no more than about 900 mm², about 1200 mm², about 1600 mm², about 2000 mm², about 2500 mm², about 3000 mm², about 3600 mm², about 4000 mm², about 4400 mm², about 4900 mm², about 5400 mm², about 5900 mm², about 6400 mm², about 6900 mm², about 7500 mm², about 8100 mm², about 8700 mm², about 9300 mm², about 10,000 mm², about 12,500 mm², about 15,000 mm², about 17,500 mm², about 20,000 mm², about 22,500 mm², about 25,000 mm², about 27,500 mm², about 30,000 mm², about 32,500 mm², about 35,000 mm², about 37,500 mm², about 40,000 mm², about 42,500 mm², about 45,000 mm², about 47,500 mm², or no more than about 50,000 mm².

The perforations may be distributed along multiple concentric circles formed around a centre point of the skin contact layer. In some embodiments, the concentric circles may be evenly spaced from one another, and/or the perforations in the same concentric circle may be evenly distributed along the circle. The concentric circles may be spaced evenly from one another.

The perforations may be distributed along multiple concentric polygons around a centre point of the adhesive skin contact layer.

The perforations may be distributed along multiple concentric decagons formed around a centre point of the adhesive skin contact layer.

The perforations may be distributed along multiple concentric squares, pentagons, hexagons, heptagons, octagons or nonagons. The concentric polygons may be evenly spaced from one another, and/or the perforations in the same concentric polygon may be evenly distributed along the polygon.

The perforations may be cut-outs (i.e., elongated perforations) formed in the adhesive skin contact layer. The adhesive skin contact layer may have a circular shape and the cut-outs may have an at least partially circular or arc shape. The cut-outs may define at least portions of multiple concentric circles. The cut-outs may have a shape at least partially extending in a parallel fashion with an outer perimeter of the adhesive skin contact layer. The adhesive skin contact layer may comprise one or more connecting portions which extend between the cut-outs and/or connecting portions of the adhesive skin contact layer separated by the cut-outs.

The plurality of perforations may be distributed evenly, or substantially evenly throughout the adhesive skin contact layer. In some embodiments, the perforations may be distributed unevenly throughout the adhesive skin contact layer, such that perforations are more concentrated in a certain portion of the adhesive skin contact layer than other portions of the adhesive skin contact layer. The perforations may be concentrated in several areas of the adhesive skin contact layer, defining clusters of perforations. Each cluster may include the same number of perforations. Each cluster may include a different number of perforations. Each of the clusters may have the same or substantially the same size and/or shape. The clusters may each have a different size and/or shape. One or more of the clusters may have a substantially same shape with each of the perforations. The clusters may have one or more shapes selected from a circle, triangle, square, rectangle, diamond, pentagon, hexagon or octagon. The perforations may be evenly or substantially evenly distributed within each of the clusters.

The adhesive skin contact layer may comprise an upper surface and a lower surface. The upper surface and/or the lower surface may be flat, or at least substantially flat, for example owing to slight raised or lowered portions in the adhesive skin contact layer surrounding each perforation, such that the adhesive skin contact layer has an even, flat profile when viewed from a side.

At least a portion of the adhesive skin contact layer may be raised, or otherwise non-flatly shaped, such that the adhesive skin contact layer has an uneven or non-planar profile when viewed from a side.

The plurality of perforations may comprise a plurality of first perforations and a plurality of second perforations.

The adhesive skin contact layer may comprise perforations with the same shape but in different orientations. For example, the plurality of second perforations may be the same shape as the plurality of first perforations but have a different orientation, for example 20 degrees, 30 degrees, 45 degrees, 60 degrees, 90 degrees, or 180 degrees rotated from the orientation of the plurality of first perforations.

The plurality of first perforations and the plurality of second perforations may have a different shape. For example, the plurality of first perforations may have a circle shape, while the plurality of second perforations may have a square shape. In some embodiments, the plurality of first perforations and the plurality of second perforations may have the same or substantially the same shape. The plurality of first perforations and the plurality of second perforations may have a different shape.

The plurality of first perforations and the plurality of second perforations may have a different size. For example, the plurality of first perforations may each have a larger diameter than each of the plurality of second perforations. In some embodiments, the plurality of first perforations and the plurality of second perforations may each have the same or substantially the same size.

Each of the plurality of first perforations and each of the plurality of second perforations may have a diameter selected from about 1.25mm, about 1.30mm, about 1.35mm, about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.10mm, about 3.20mm, about 3.30mm, about 3.40mm, about 3.50mm, about 3.60mm, about 3.70mm, about 3.80mm, about 3.90mm, about 4.00mm, about 4.10mm, about 4.20mm, about 4.30mm, about 4.40mm, about 4.50mm, about 4.60mm, about 4.70mm, about 4.80mm, about 4.90mm, or about 5.00mm.

Each of the plurality of first perforations and each of the plurality of second perforations may have a diameter selected from at least about 1.25mm, about 1.30mm, about 1.35mm, about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.10mm, about 3.20mm, about 3.30mm, about 3.40mm, about 3.50mm, about 3.60mm, about 3.70mm, about 3.80mm, about 3.90mm, about 4.00mm, about 4.10mm, about 4.20mm, about 4.30mm, about 4.40mm, about 4.50mm, about 4.60mm, about 4.70mm, about 4.80mm, about 4.90mm, or at least about 5.00mm.

Each of the plurality of first perforations and each of the plurality of second perforations may have a diameter selected from no more than about 1.25mm, about 1.30mm, about 1.35mm, about 1.40mm, about 1.45mm, about 1.50mm, about 1.55mm, about 1.60mm, about 1.65mm, about 1.70mm, about 1.75mm, about 1.80mm, about 1.85mm, about 1.90mm, about 1.95mm, about 2.00mm, about 2.05mm, about 2.10mm, about 2.15mm, about 2.20mm, about 2.25mm, about 2.30mm, about 2.35mm, about 2.40mm, about 2.45mm, about 2.50mm, about 2.55mm, about 2.60mm, about 2.65mm, about 2.70mm, about 2.75mm, about 2.80mm, about 2.85mm, about 2.90mm, about 2.95mm, about 3.00mm, about 3.10mm, about 3.20mm, about 3.30mm, about 3.40mm, about 3.50mm, about 3.60mm, about 3.70mm, about 3.80mm, about 3.90mm, about 4.00mm, about 4.10mm, about 4.20mm, about 4.30mm, about 4.40mm, about 4.50mm, about 4.60mm, about 4.70mm, about 4.80mm, about 4.90mm, or no more than about 5.00mm.

The plurality of first perforations may be arranged in a border portion of the adhesive skin contact layer. The plurality of second perforations may be arranged in a central portion of the adhesive skin contact layer. The central portion may be surrounded by the border portion. The plurality of first perforations may each have a larger diameter than each of the plurality of second perforations.

In some embodiments, the plurality of first perforations and the plurality of second perforations may be distributed in a different configuration. For example, the plurality of first perforations may be distributed in multiple columns and the first perforations of the adjacent columns may be staggered to each other, such that the plurality of first perforations are distributed in a triangular packing layout, while the plurality of second perforations are distributed around each of the first perforations.

The invention according to the first aspect may of course individually include any one or more of the features, optional or otherwise, of the invention according to any other aspect.

According to a second aspect of the present invention, there is provided a method for forming a plurality of perforations in the adhesive skin contact layer of the negative pressure wound dressing, the method comprising:
a. Providing an adhesive skin contact layer; and
b. Forming a plurality of perforations in the adhesive skin contact layer by mechanical punch perforating.

The adhesive skin contact layer may have a weight of between 40gsm and 250gsm.

The adhesive skin contact layer may be a perforated mesh. The perforated mesh may comprise perforations each having a diameter of between 1.25mm and 5.00mm.

The invention according to the second aspect may of course individually include any one or more of the features, optional or otherwise, of the invention according to any other aspect. For example, the adhesive skin contact layer may be the adhesive skin contact layer as defined in the first aspect, optionally including any optional feature set out above, and the wound dressing may be the wound dressing of the first aspect, optionally including any optional feature set out above.

According to an further aspect of the invention, there is provided a negative pressure wound exudate management system comprising the wound dressing of any previous aspect, and further comprising:
a source of negative pressure; and
a conduit or tube for providing negative pressure to the wound dressing via an internal lumen of the conduit or tube and an opening in the wound dressing.

The source of negative pressure may comprise a pump for generating negative pressure and the tube connects the pump and wound dressing.

The source of negative pressure may further comprise a one-way valve in-line between the pump and the wound dressing to maintain a negative pressure within the wound dressing when the pump is disconnected from the tube. Normal operation of the pump may include the generation of negative pressure, alternating periods of negative pressure generation, and/or no generation of negative pressure.

The one-way valve may be connected to one of the pump and the tube.

The opening in the wound dressing may be in a backing layer of the wound dressing. The backing layer of the wound dressing may be a bacterial and viral barrier layer which preferably resists the ingress of liquid and air but allows moisture vapour transmission. In this way the backing layer enhances the overall fluid handling capacity of the wound dressing by allowing for the escape of moisture vapour through the backing layer while enabling the application of negative pressure to the wound. The backing layer may be a layer having a Moisture Vapor Transmission Rate (MVTR) of at least about 10,000 g/m² per 24 hours or in the range of from about 10,000g/m² to about 50,000g/m² per 24 hours as measured by the method described in BS EN 13726-22002 "Test methods for primary wound dressings Part 2 Moisture vapour transmission rate of permeable film dressings". The backing layer may be in the form of a film of polyurethane.

The conduit or tube may comprise a proximal end having an opening. The proximal end of the conduit or tube may be adhered around the opening in the backing layer of the wound dressing such that the opening in the backing layer is in fluid communication with the opening in the proximal end of the conduit or tube. The adhesion between the proximal end of the conduit or tube and the opening in the backing layer may for an airtight, or substantially airtight, seal between the conduit or tube and the backing layer.

The backing layer may be provided with a port, or airway for connection to the conduit. The port or airway may be located in that part of the backing layer that overlies the absorbent structure but towards the periphery of the absorbent structure so that it is not directly in vertical alignment with the centre of the wound dressing (or the wound when in use). This assists in the spread of wound exudate across the full extent of the absorbent layer.

The invention according to the eighth aspect may of course individually include any one or more of the features, optional or otherwise, of the invention according to any other aspect.

### Detailed Description of the Invention

In order that the invention may be more clearly understood one or more embodiments thereof will now be described, by way of example only, with reference to the accompanying drawings, of which:
- Figure 1: is an example embodiment of part of an adhesive skin contact layer for use with a negative pressure wound dressing;
- Figure 2: is an expanded view of section A of Figure 1;
- Figure 3: is an exploded view of a first embodiment of a negative pressure wound dressing;
- Figure 4: is a plan view of a further example embodiment of a negative pressure wound dressing;
- Figure 5: is an exploded view of the negative pressure wound dressing of Fig. 4;
- Figure 6: is a plan view of an example embodiment of a backing layer for use with the embodiment shown in Fig. 4;
- Figure 7: is a perspective view of the example backing layer shown in Fig. 6;
- Figure 8: is a plan view of an example embodiment of an absorbent structure for use with the embodiment shown in Fig. 4;
- Figure 9: is a side view of the example absorbent structure shown in Fig. 8;
- Figure 10: is a rear view of the example absorbent structure shown in Fig. 8;
- Figure 11: is a cross-section view along line A-A shown in Fig. 8;
- Figure 12: is a perspective view of the example absorbent structure shown in Fig. 8;
- Figure 13: is a plan view of a lower surface of an example embodiment of an adhesive skin contact layer for use with the embodiment shown in Fig. 4;
- Figure 14: is a perspective view of the example adhesive skin contact layer shown in Fig. 13;
- Figure 15: is a further plan view of the example embodiment of the negative pressure wound dressing shown in Fig. 4;
- Figure 16: is a cross-section view along line B-B shown in Fig. 15;
- Figure 17: is a perspective view of the example embodiment of the negative pressure wound dressing shown in Fig. 4; and
- Figure 18: is a perspective view of an example embodiment of a release liner for use with the embodiment shown in Fig. 4.

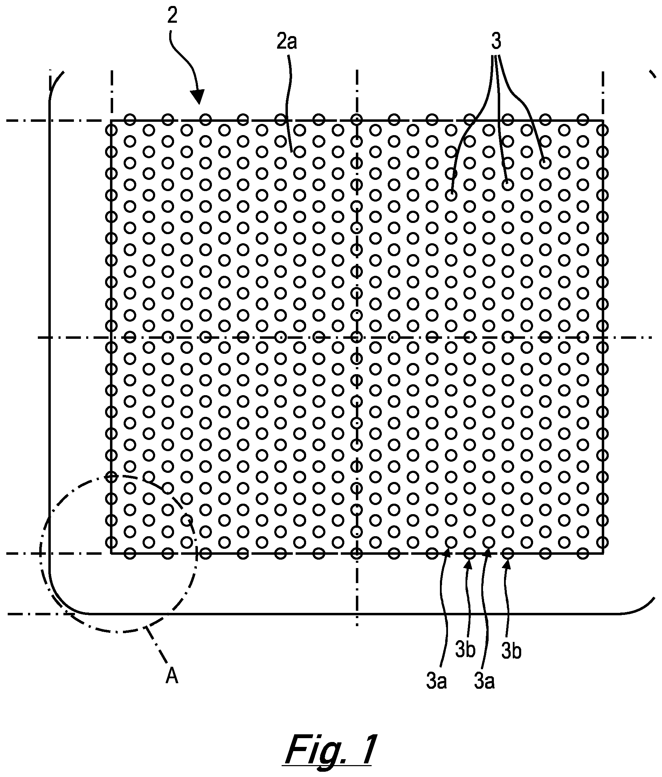

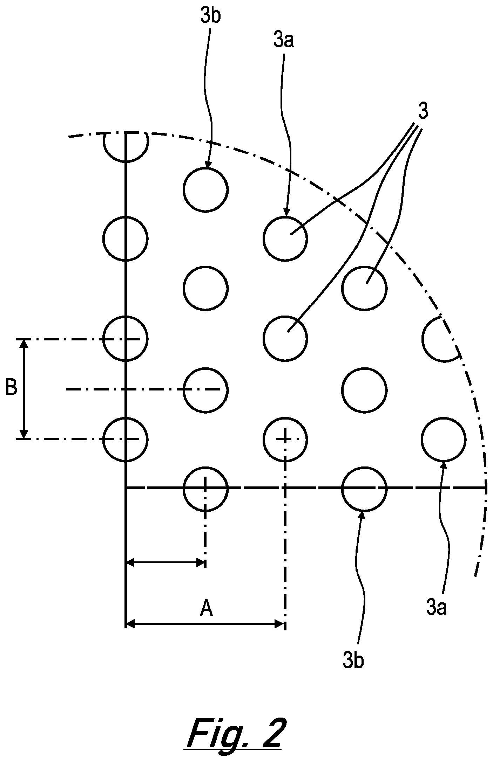

With reference to Figures 1 and 2, there is shown an adhesive skin contact layer 2 for use with a negative pressure wound dressing.

The adhesive skin contact layer assists in securing the wound dressing to the skin of a patient, in particular to the peri-wound skin adjacent to a wound. The adhesive skin contact layer 2 may have a weight of 200gsm which provides for a malleable, gelled structure so, in the event of an air leak, the layer can seal the leak more easily than if the layer had a weight below 100gsm, in particular below 40gsm. The adhesive skin contact layer 2 is a perforated mesh comprising a plurality of perforations 3 arranged in a plurality of rows. In the present embodiment, 95% of the perforations 3 are arranged in a triangle packed layout. The triangle packed layout comprises adjacent rows 3a, 3b of perforations 3. Row 3a is arranged offset from row 3b. The perforations 3 in row 3a are not arranged adjacent to the perforations 3 in the adjacent row 3b but instead the perforations 3 in row 3a are arranged adjacent to the gap between perforations 3 in row 3b or adjacent to part of the gap and part of a perforation 3 in row 3b.

Each perforation 3 comprises an opening, which in this embodiment is in the shape of a circle. In this embodiment, the size of each perforation 3 is defined by the diameter of the circle. However, the person skilled in the art will understand that the size of the opening may be defined depending on the shape of the opening. In some embodiments, the size of a circle is the diameter. In other embodiments, the size of an oval is the longer diameter. In further embodiments, the size of a hexagon is the longest diagonal; and the size of a triangle, a square, a rectangle, an octagon or any other polygon perforation may be the longest side. In the present embodiment, each perforation 3 has a diameter of 2.20mm and are therefore of sufficient size to provide breathability to the wound dressing in the event that some of the perforations reduce in diameter after their formation, for example by the adhesive entering the opening of a perforation. As such, the adhesive skin contact layer maintains an environment optimised for wound healing. Moreover, each perforation 3 is a through hole in the adhesive skin contact layer 2 which can enable fluid to flow through the layer 2. The adhesive skin contact layer 2, therefore, helps to prevent tissue ingrowth into the other material of the wound dressing.

The perforations 3 have a pitch (i.e., the distance between the centre point of a perforation and the centre point of an adjacent perforation) in a first direction (shown as 'A' in Figure 2) of 8.0mm, and a pitch in a second direction (shown as 'B' in Figure 2) of 5.6mm. Advantageously, the pitch of the perforations 3 has a ratio of perforation : no-perforation which provides efficient moisture evaporation, prevents pooling and strongly adheres to the peri-wound skin of the patient. Surprisingly, and advantageously, tests have shown that the adhesive skin contact layer 2 remains adhered to a patient for at least seven days.

The total area of the perforations 3 may be 2100mm² and the total volume of the perforations may be 2100mm³. The degree of perforation may be 10.66% of the area of the adhesive skin contact layer 2. The perforations 3 are distributed evenly throughout the adhesive skin contact layer 2. The perforations 3 may be formed by a mechanical punch perforating process which does not result in the formation of a doughnut or volcano structure around the perforations after formation of the perforations 3. The perforations may be formed by mechanical punch perforating the adhesive skin contact layer 2 comprising a release liner (not shown) covering at least one surface of the adhesive skin contact layer 2.

The adhesive skin contact layer 2 has a thickness of 1.0mm and a width and length (measured from the periphery of the adhesive skin contact layer 2) each of 130mm. The adhesive skin contact layer 2 has an outer periphery in the shape of a square. The adhesive skin contact layer 2 comprises an upper surface 2a and a lower surface 2b. The lower surface 2b contacts the dermal surface in use.

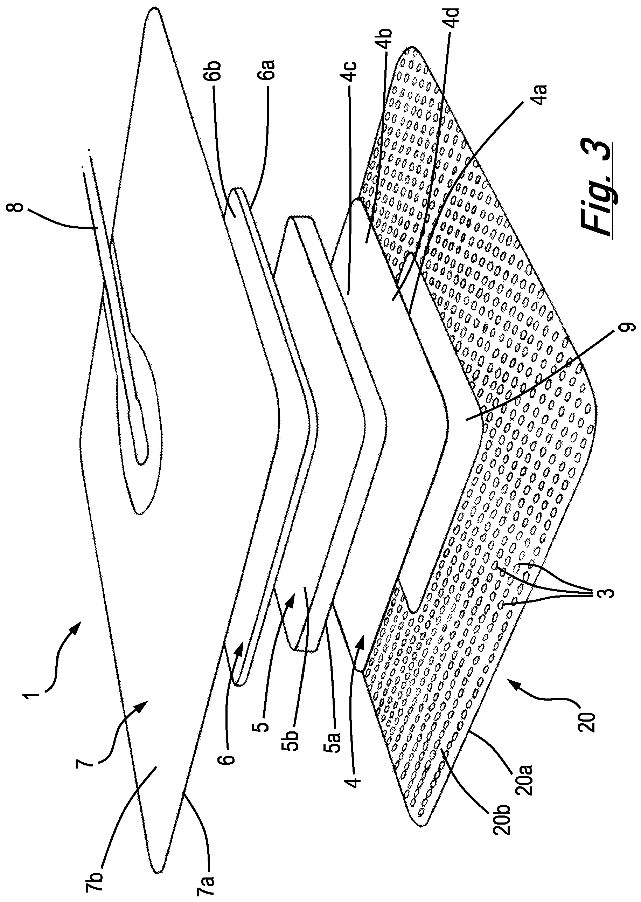

With reference to Figure 3, there is shown a negative pressure wound dressing 1.

The wound dressing 1 comprises an adhesive skin contact layer 20 (which is preferably the adhesive skin contact layer 2 described with reference to Figures 1 and 2), a wound contact layer 4, a transmission layer 5, a superabsorbent layer 6, a backing layer 7 and an airway 8.

The adhesive skin contact layer 20 may have a perimetral shape with a central window 9, therefore, the adhesive skin contact layer 20 may be an adhesive skin contact border layer 20. The border layer 20 may comprise an outer periphery which defines the outer shape of the border layer 20, which, in this embodiment, is a square. The border layer 20 may comprise an inner periphery which defines the shape of the window 9. The window 9 may be of the same, or similar shape, to that of the outer periphery of the border layer 20.

The window 9 is a square through hole in the adhesive skin contact layer 20. The window 9 is located centrally relative to the periphery of the adhesive skin contact layer 20. Each side of the window 9 is arranged parallel to each of the corresponding sides of the adhesive skin contact layer 20. The window may have an area of 12,500mm2. The skilled person will appreciate that the window 9 may be enlarged by any suitable means, for example by cutting the adhesive skin contact layer 20. The window 9 in the adhesive skin contact layer 20 enables the wound of a patient to contact, or be in fluid communication with, other layers of the wound dressing 1, for example the wound contact layer 4, when the wound dressing 1 is applied to the skin of a patient.

The adhesive skin contact layer 20 comprises an upper surface 20b and a lower surface 20a. The lower surface 20a contacts the dermal surface in use. **In** use, the lower surface 20a of the adhesive skin contact layer 20 is detachably adhered to a dermal surface (not shown) of a patient such that the wound site (not shown) is located within the window 9. As such, the adhesive skin contact layer 20 is not in direct contact with the wound site but does wholly surround it. Thus, the adhesive skin contact layer 20 is in contact with intact peri-wound skin only. The upper surface 20b may be outwardly facing away from the patient's body.

A removable cover or "release layer" (not shown) may be adhered to the lower surface 20a of the adhesive skin contact layer 20. The removable cover, which may comprise folded grip sections, is removable from the lower surface 20a of the adhesive skin contact layer 20. Thus, the removable cover protects the adhesive skin contact layer 20 when the removable cover is adhered to the adhesive skin contact layer 20, but when the removable cover is removed from the lower surface 20a of the adhesive skin contact layer 20 for use of the wound dressing 1, the lower surface 20a is exposed and able to releasably secure the wound dressing 1 to the skin of a patient.

The wound contact layer 4, the transmission layer 5 and the superabsorbent layer 6 form an absorbent structure arranged between the adhesive skin contact layer 20 and the backing layer 7.

The backing layer 7 may comprise a first surface 7a and a second surface 7b. In use, the first surface 7a may be inwardly facing toward the patient's body, and the second surface 7b may be outwardly facing away from the patient's body.

The absorbent structure 4, 5, 6 may comprise a first surface 4a and a second surface 6b. The first surface 4a of the absorbent structure 4, 5, 6 may be adjacent, and in contact with, the upper surface 20b of the adhesive skin contact layer 20. The peripheral edge of the second surface 6b of the absorbent structure 4, 5, 6 may be adjacent, and in contact with, the first surface 7a of the backing layer 7.

The wound contact layer 4 may comprise a first surface 4a and a second surface 4b. In use, the first surface 4a may be inwardly facing toward the patient's body, and the second surface 4b may be outwardly facing away from the patient's body. The first surface 4a of the wound contact layer 4 may contact the wound when the wound dressing 1 is adhered to the skin adjacent to the wound (i.e., when in use). The first surface 4a of the wound contact layer 4 is substantially aligned with the window 9 of the adhesive skin contact layer 20. As such, in use, the first surface 4a contacts the wound through the window 9. The second surface 4b of the wound contact layer 4 may be adjacent, and in contact with, a first surface 5a of the transmission layer 5 or a first surface 6a of the superabsorbent layer 6. In the described embodiment, the second surface 4b of the wound contact layer 4 is adjacent, and in contact with, the first surface 5a of the transmission layer 5.

The transmission layer 5 may comprise a first surface 5a and a second surface 5b. **In** use, the first surface 5a may be inwardly facing toward the patient's body, and the second surface 5b may be outwardly facing away from the patient's body. The first surface 5a of the transmission layer 5 may be adjacent, and in contact with, the second surface 4b of the wound contact layer 4 or a second surface 6b of the superabsorbent layer 6. The second surface 5b of the transmission layer 5 may be adjacent, and in contact with, a first surface 6a of the superabsorbent layer 6 or the first surface 7a of the backing layer 7. In the described embodiment, the first surface 5a of the transmission layer 5 is adjacent, and in contact with, the second surface 4b of the wound contact layer, and the second surface 5b of the transmission layer 5 is adjacent, and in contact with, the first surface 6a of the superabsorbent layer 6.

The superabsorbent layer 6 may comprise a first surface 6a and a second surface 6b. In use, the first surface 6a may be inwardly facing toward the patient's body, and the second surface 6b may be outwardly facing away from the patient's body. The first surface 6a of the superabsorbent layer 6 may be adjacent, and in contact with, the second surface 4b of the wound contact layer 4 or the second surface 5b of the transmission layer 5. The second surface 6b of the superabsorbent layer 6 may be adjacent, and in contact with, the first surface 5a of the transmission layer 5 or the first surface 7a of the backing layer 7. In the described embodiment, the first surface 6a of the superabsorbent layer 6 is adjacent, and in contact with, the second surface 5b of the transmission layer 5, and the second surface 6b of the superabsorbent layer 6 is adjacent, and in contact with, the first surface 7a of the backing layer 7.

The adhesive skin contact layer 20 may radially overlap the absorbent structure 4, 5, 6. The adhesive skin contact layer 20 may comprise an interior portion adjacent to and surrounding the window 9, and an exterior portion radially outwardly from the interior portion. The interior portion and the exterior portion of the first surface 20a of the adhesive skin contact layer 20 may contact and adhere to the skin of a patient in use. The interior portion of the second surface 20b of the adhesive skin contact layer 20 may be adjacent, and in contact with, a portion of the first surface 4a of the wound contact layer 4. The exterior portion of the second surface 20b of the adhesive skin contact layer 20 may be adjacent, and joined with, a peripheral portion of the first surface 7a of the backing layer 7. Beneficially, this helps to prevent wound exudate escaping from the confines of the wound dressing which would be unhygienic and cause discomfort for the user. Moreover, this also helps maintain a negative pressure at the wound site.

The exterior portion of the second surface 20b of the adhesive skin contact layer 20 may be joined to the peripheral portion of the first surface 7a of the backing layer 7 by heat lamination, adhesive, welding or stitching.

The exterior portion of the second surface 20b of the adhesive skin contact layer 20 may extend radially beyond the periphery of the absorbent structure by about 27.5 mm.

At least two of the wound contact layer 4, the transmission layer 5 and the superabsorbent layer 6 may be laminated together. In the described embodiment, each of the wound contact layer 4, the transmission layer 5 and the superabsorbent layer 6 are laminated together. Advantageously, this means that the absorbent structure of the present invention is manufactured without requiring any relatively rigid dressing and binding components. Thus, the present invention provides an absorbent structure which is flexible, therefore benefits user comfort, and which is cheaper to manufacture than absorbent structures and, therefore negative pressure wound dressings, of the prior art. Moreover, lamination does not require any complex equipment or manufacturing techniques. Thus, the present invention is simple to manufacture.

Each of the wound contact layer 4, the transmission layer 5 and the superabsorbent layer 6 may be laminated together by a scatter-coat adhesive. The scatter-coat adhesive may be a hot melt, scatter-coat adhesive. In the described embodiment, the scatter-coat adhesive is polycaprolactone and has the following properties:
- a melt flow index of between 5.2 g/10min and 11.3 g/10min, as tested with 2.16 kg, 1" PVC die at 160°C;
- a maximum water content of 0.35%;
- a mean molecular weight of 50,000;
- a melting point of 60°C; and
- a solubility parameter of 9.34 cal/cm3 to 9.43 cal/cm3.

The scatter-coat adhesive may comprise particles of which at least 98% have a particle size of 0.6mm.

The wound contact layer 4, the transmission layer 5 and the superabsorbent layer 6 may be laminated together by a laminator having the following processing properties:
- Heating zone temperature of 125°C;
- Cooling zone temperature of 25°C;
- Web tension (for each unwind reel) of 125 N;
- Crush roller pressure of 1000 N; and
- Web speed of 10 m/min.

The wound contact layer 4 further comprises a central region 4c and a peripheral region 4d. The central region 4c is substantially square and is bordered on each side by the peripheral region 4d.

In the described embodiment, the first surface 4a, which contacts the wound in use, may comprise warp stitches (longitudinal to the plane of the wound contact layer 4) only, i.e., the first surface 4a does not comprise any weft stitches (transverse to the plane of the wound contact layer 4). Advantageously, this means that the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced and an environment optimised for wound healing is maintained at the wound site.

The warp stitches of the first surface 4a may have a pitch of 6.6mm and a stitch density of 7.5 stitches /cm.

The second surface 4b, which is laminated to the first surface 5a of the transmission layer 5, comprises warp stitches and weft stitches. Each of the warp stitches and the weft stitches of the second surface 4b have a pitch of 6.6mm and a stitch density of 7.5 stitches /cm.

In other embodiments, the central region 4c of the first surface 4a may comprise warp stitches only and the peripheral region 4d of the first surface 4a may comprise warp and weft stitches.

The wound contact layer 4 may have a weight of 100gsm and a thickness of 3.0 mm.

Between the first surface 4a and the second surface 4b, which are joined about their periphery, the wound contact layer 4 may comprise a plurality of gel-forming fibres which, upon the uptake of wound exudate, become moist, slippery or gelatinous. Specifically, in this embodiment, the gel-forming fibres are hygroscopic fibres which upon the uptake of wound exudate become gelatinous. The gel-forming fibres may be of the type which retain their structural integrity on absorption of exudate. In the present embodiment, the fibres are sodium carboxymethylcellulose fibres having a degree of substitution of 0.30 carboxymethyl groups per glucose unit, as measured by IR spectroscopy (as defined in WO00/01425). The absence of weft stitches at the first surface 4a means that a greater amount of gel-forming fibres, as described above, are exposed at the first surface and, therefore, in contact with the wound. As such, the wound contact layer is higher gelling, i.e., has a significantly greater absorbency capacity and is more retentive, than gel-forming fibres of the prior art. As such, the wound contact layer 4 absorbs a greater amount of wound exudate. Thus, pooling of wound exudate at the wound site is prevented, or at least significantly reduced.

The sodium carboxymethylcellulose fibres are made by carboxymethylating a non-woven cellulosic fabric having an absorbency of 18g/g of Solution A (sodium/calcium chloride solution), as measured by the method described in BS EN 13726-1 (2002) "Test methods for primary wound dressings", section 3.2 "Free swell absorptive capacity". The carboxymethylation is performed by contacting the fabric with an alkali and a carboxymethylating agent such as chloroacetic acid in a combined alcohol and aqueous system. The cellulosic fabric comprises solely of cellulosic fibre and comprises continuous filament yarn.

The gel-forming fibres have an absorbency of 2 grams 0.9% saline solution per gram of fibre (as measured by the free swell method).

The wound contact layer 4 is stitched using lyocell yarn (produced under the trade name Tencel^{™} yarn).

The transmission layer 5 is substantially square, as is the wound contact layer 4 and the superabsorbent layer 6, and has a thickness of 1.8mm and is made of a polyurethane foam. The transmission layer 5 may have a density of between 26 Kg/M3 and 28 Kg/M3 and a tensile strength of 150 kPa. The transmission layer 5 may have an elongation at break value of 300% and a cell count of between 17 and 26 /cm. Moreover, the transmission layer 5 may have a 40% CLD hardness of between 2.5 and 4.5 kPa.

The superabsorbent layer 6 comprises an upper layer having the second surface 6b, and a lower layer having the first surface 6a. Between the upper layer and the lower layer, there is enclosed a first material comprising non-woven fibres, an absorbent material and a hot melt binder.

The hot melt binder may be present in an amount of 70 g/m². The hot melt binder may be a copolymer and, in the described embodiment, may be ethylene-vinyl acetate (EVA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

The absorbent material may be a superabsorbent material, capable of absorbing wound exudate while allowing the passage of fluid through it. The superabsorbent material may be sodium polyacrylate and in the form of a powder which, advantageously, increases the surface area to volume ratio of the superabsorbent material, therefore enhancing the absorbent property of the material.

The non-woven fibres may be cellulose fibres. The cellulose non-woven fibres may be arranged as a cellulosic non-woven matrix of fibres and may be a support onto which the absorbent material may be incorporated, for example, in the described embodiment, the absorbent material is dispersed upon the cellulosic non-woven matrix. The absorbent material may be dispersed evenly, or substantially evenly, throughout or upon the non-woven fibres, for example the cellulosic non-woven matrix.

The upper layer and the lower layer of the superabsorbent layer 6 may each be a cellulosic fabric layer. The upper fabric layer and the lower fabric layer may be joined around their entire periphery to enclose the first material.

The superabsorbent layer 6 may comprise a plurality of fenestrations (not shown). Each of the plurality of fenestrations may be openings in the form of slits in the superabsorbent layer 6. Each fenestration may have a length equal to 80% of the length of the superabsorbent layer 6. Each of the upper layer and the lower layer may comprise a plurality of fenestrations. The fenestrations may be arranged in a direction planar to a longitudinal axis and transverse to a longitudinal axis of the superabsorbent layer 6. Advantageously, this arrangement aids in managing a flow of exudate through the superabsorbent layer 6 of the wound dressing 1. The fenestrations encourage or enable wound exudate to travel laterally on the superabsorbent layer 6, as opposed to axially, and assist in negative pressure transmission through the superabsorbent layer 6.

The superabsorbent layer 6 may have a basis weight of 460 g/m2, a thickness of 2.55 mm and a density of 0.255 g/cm3. Moreover, the superabsorbent layer 6 may have a tensile strength (dry) of 45 N/50mm, an absorbent capacity of 25 g/g 10min, and an absorbency under compression capacity of 0.85 g/cm2.

Each of the wound contact layer 4, transmission layer 5 and superabsorbent layer 6 are substantially planar.

In use, the wound dressing 1 forms part of a negative pressure wound exudate management system comprising the wound dressing 1 and further comprising a source of negative pressure (not shown) and a coupling member (for example, airway 8) for providing negative pressure to the wound dressing from an internal lumen of a conduit or tube (not shown) and through an opening (not shown) in the wound dressing. The source of negative pressure comprises a pump for generating negative pressure and the tube connects the pump and wound dressing 1 via the airway 8. In the present embodiment, the source of negative pressure may comprise a one-way valve (not shown) in-line between the pump and the wound dressing 1 to maintain a negative pressure within the wound dressing 1 when the pump is disconnected from the tube.

In use, the adhesive skin contact layer 20 is adhered to the intact peri-wound skin around a wound. The wound is located within the window 9 of the adhesive skin contact layer 20. The wound dressing 1 is connected to a source of negative pressure before or after application of the dressing to the wound. Upon leaving the wound, wound exudate contacts the first surface 4a of the wound contact layer 4 and wicks upwardly, away from the wound site, toward the transmission layer 5. The high gelling ability of the wound contact layer 4 facilitates absorption and retention of the wound exudate. Lamination of the wound contact layer 4, transmission layer 5 and superabsorbent layer 6 means each of these layers form a consolidated, absorbent and retentive structure. The wound exudate wicks from the upper surface 4b of the wound contact layer 4 to the transmission layer 5. Lateral movement of the exudate occurs in the transmission layer 5 to spread the wound exudate across the surface area of the dressing. The exudate then wicks upwardly to the superabsorbent layer 6 where it is absorbed and retained prior to being transmitted out of the dressing 1 via vapour transmission through the backing film 7.

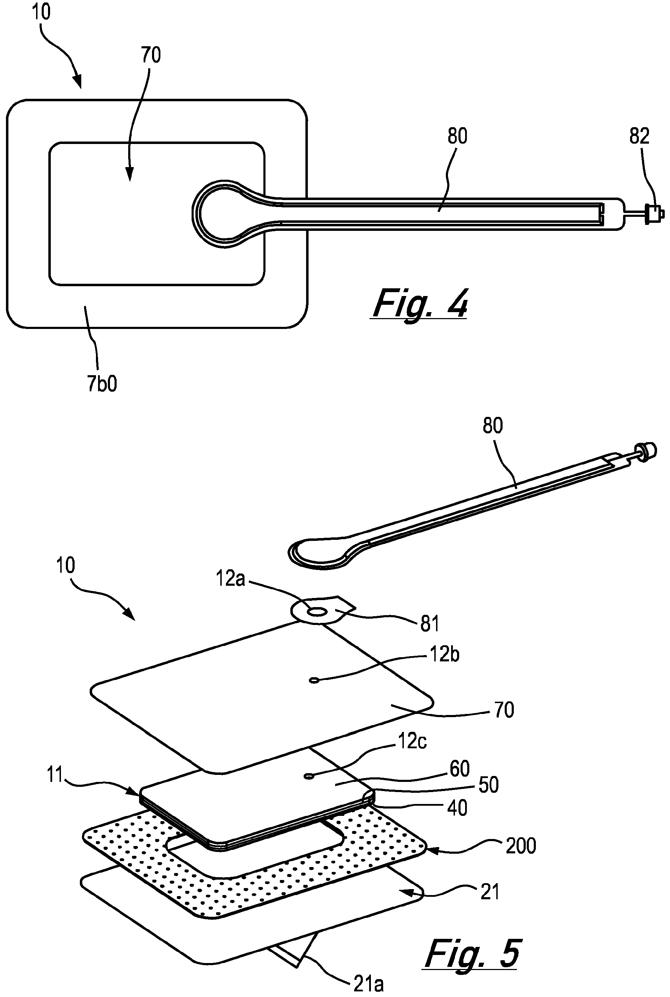

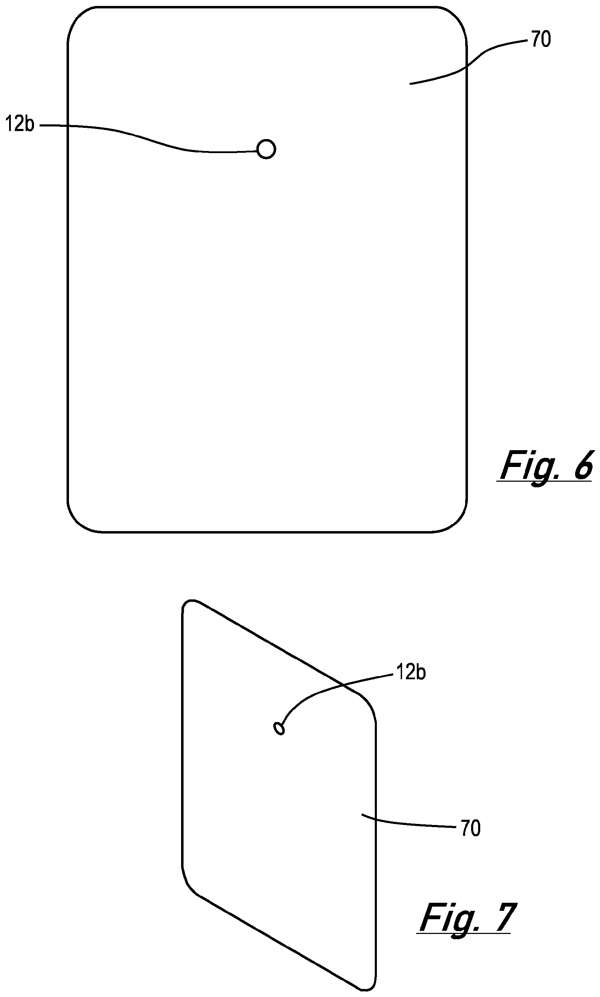

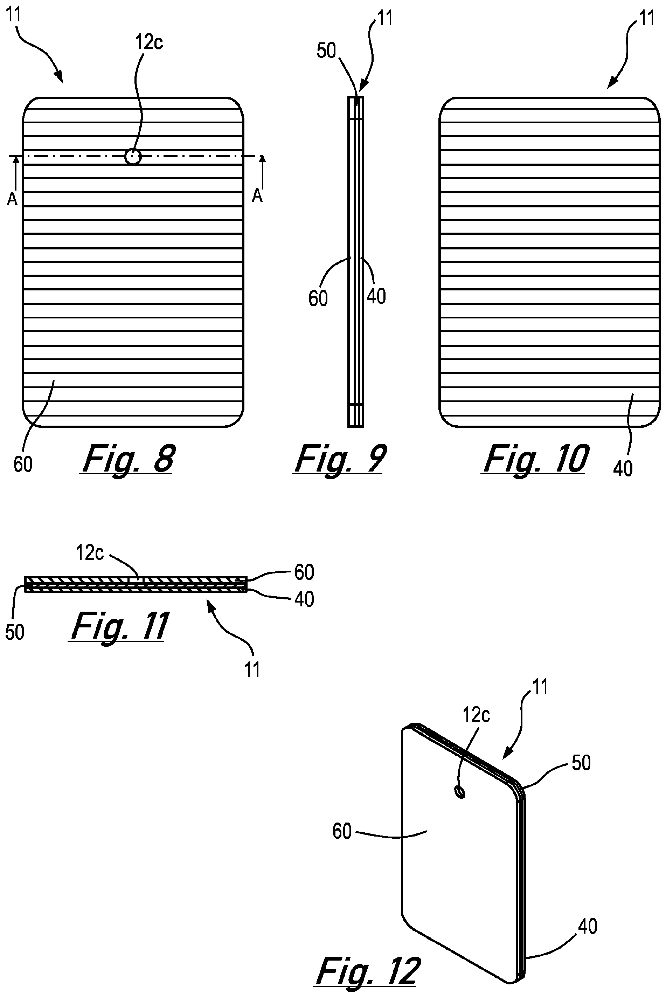

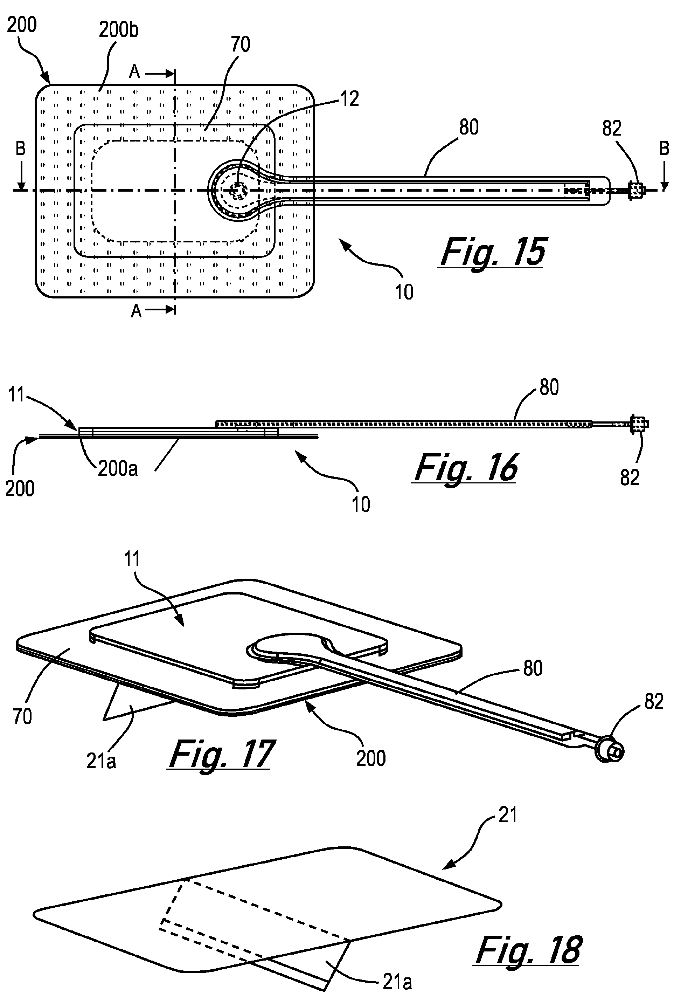

With reference to Figures 4 to 18, there is shown a further example embodiment of a negative pressure wound dressing 10.

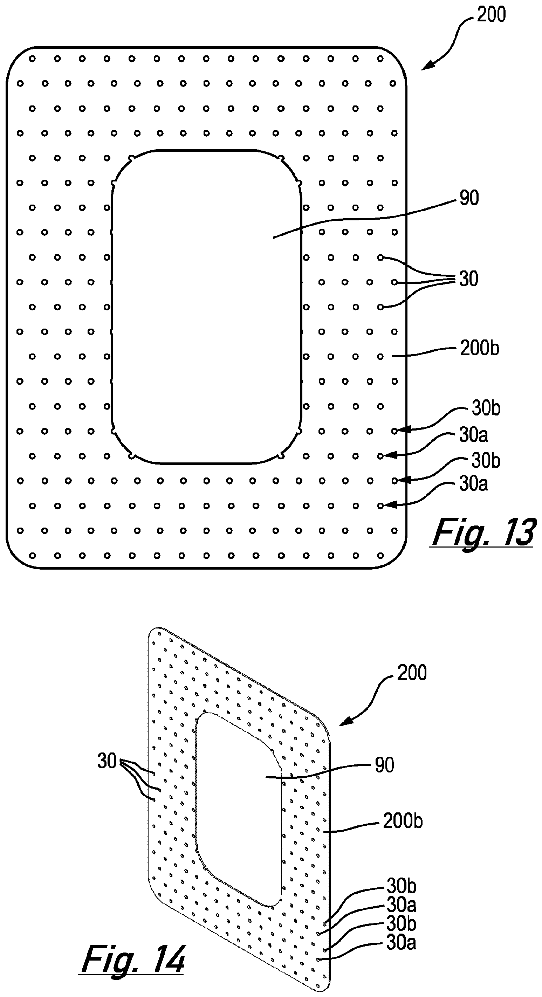

The wound dressing 10 comprises an adhesive skin contact layer 200 (which is preferably the adhesive skin contact layer 200 described with reference to Figures 13 and 14), a wound contact layer 40, a transmission layer 50, a superabsorbent layer 60, a backing layer 70 and an airway 80. The wound contact layer 40, transmission layer 50 and superabsorbent layer 60 together form an absorbent structure 11 arranged between the adhesive skin contact layer 200 and the backing layer 70.

The adhesive skin contact layer 200 assists in securing the wound dressing 10 to the skin of a patient, in particular to the peri-wound skin adjacent to a wound. The adhesive skin contact layer 200 may have a weight of 200gsm which provides for a malleable, gelled structure so, in the event of an air leak, the layer can seal the leak more easily than if the layer had a weight below 100gsm, in particular below 40gsm. With reference to Figs. 13 and 14, the adhesive skin contact layer 200 may be a perforated mesh comprising a plurality of perforations 30 arranged in a plurality of rows. In the present embodiment, 95% of the perforations 30 are arranged in a triangle packed layout. The triangle packed layout comprises adjacent rows 30a, 30b of perforations 30. Row 30a is arranged offset from row 30b. The perforations 30 in row 30a are not arranged adjacent to the perforations 30 in the adjacent row 30b but instead the perforations 30 in row 30a are arranged adjacent to the gap between perforations 30 in row 30b or adjacent to part of the gap and part of a perforation 30 in row 30b.

Each perforation 30 comprises an opening, which in this embodiment is in the shape of a circle. In this embodiment, the size of each perforation 30 is defined by the diameter of the circle. However, the person skilled in the art will understand that the size of the opening may be defined depending on the shape of the opening. In some embodiments, the size of a circle is the diameter. In other embodiments, the size of an oval is the longer diameter. In further embodiments, the size of a hexagon is the longest diagonal; and the size of a triangle, a square, a rectangle, an octagon or any other polygon perforation may be the longest side. In the present embodiment, each perforation 30 has a diameter of 2.20mm and are therefore of sufficient size to provide breathability to the wound dressing in the event that some of the perforations reduce in diameter after their formation, for example by the adhesive entering the opening of a perforation. As such, the adhesive skin contact layer maintains an environment optimised for wound healing. Moreover, each perforation 30 is a through hole in the adhesive skin contact layer 200 which can enable fluid to flow through the layer 200. The adhesive skin contact layer 200, therefore, helps to prevent tissue ingrowth into the other material of the wound dressing.

The perforations 30 may have a pitch (i.e., the distance between the centre point of a perforation and the centre point of an adjacent perforation) in a first direction (shown as 'A' in Figure 2 and applicable to the perforations 30 shown in Figs. 13 and 14) of 8.0mm, and a pitch in a second direction (shown as 'B' in Figure 2 and applicable to the perforations 30 shown in Figs. 13 and 14) of 5.6mm. Advantageously, the pitch of the perforations 30 has a ratio of perforation : no-perforation which provides efficient moisture evaporation, prevents pooling and strongly adheres to the peri-wound skin of the patient. Surprisingly, and advantageously, tests have shown that the adhesive skin contact layer 200 remains adhered to a patient for at least seven days.

The total area of the perforations 30 may be 2100mm² and the total volume of the perforations may be 2100mm³. The degree of perforation may be 10.66% of the area of the adhesive skin contact layer 200. The perforations 30 are distributed evenly throughout the adhesive skin contact layer 200. The perforations 30 may be formed by a mechanical punch perforating process which does not result in the formation of a doughnut or volcano structure around the perforations after formation of the perforations 30. The perforations may be formed by mechanical punch perforating the adhesive skin contact layer 200 comprising a release liner (not shown) covering at least one surface of the adhesive skin contact layer 200.

The adhesive skin contact layer 200 may have a thickness of 1.0mm and a width and length (measured from the periphery of the adhesive skin contact layer 200) each of 130mm. The adhesive skin contact layer 200 has an outer periphery in the shape of a square. The adhesive skin contact layer 200 comprises an upper surface 200a and a lower surface 200b. The lower surface 200b contacts the dermal surface in use.

The adhesive skin contact layer 200 may have a perimetral shape with a central window 90, therefore, the adhesive skin contact layer 200 may be an adhesive skin contact border layer 200. The border layer 200 may comprise an outer periphery which defines the outer shape of the border layer 200, which, in this embodiment, is a rectangle. The border layer 200 may comprise an inner periphery which defines the shape of the window 90. The window 90 may be of the same, or similar shape, to that of the outer periphery of the border layer 200. In other embodiments, the window may have a different to that of the outer periphery of the border layer 200. For example, in embodiments comprising a border layer having an outer periphery which is square, the window may be, for example, a square or a rectangle. In embodiments, comprising a border layer having a square outer periphery and a window which is a rectangle, there may be greater overlap between the absorbent structure and the adhesive skin contact layer, therefore, allowing for a greater surface area for adhesion between the adhesive skin contact layer and the absorbent structure.

In this embodiment, the window 90 has a perimeter which is a rectangle, therefore, the length of the transverse sides of the window is less than the length of the longitudinal sides of the window 90. The window 90 is a rectangle through-hole in the adhesive skin contact layer 200. The shape of the window 90 provides for greater overlap of the absorbent structure 11 and the adhesive skin contact layer 200, especially at the corresponding corner portion of the absorbent structure 11 and the adhesive skin contact layer 200. This is beneficial not least because it allows for a greater surface area for adhesion between the adhesive skin contact layer 200 and the absorbent structure 11, therefore, providing greater structural integrity to the wound dressing 10. The greater structural integrity means that the absorbent structure 11 maintains its position within the wound dressing 10 with reduced possibility of the absorbent structure 11 becoming dislodged from its position within the wound dressing 10 when in use, in particular when in use when the wound dressing 10 is wet with exudate or other fluids.

The window 90 is located centrally relative to the periphery of the adhesive skin contact layer 200. Each side of the window 90 is arranged parallel to each of the corresponding sides of the adhesive skin contact layer 200. Each longitudinal side of the window 90 may have a length of about 210mm and each traverse side of the window 90 may have a length of about 160mm. The window may have an area of about 33,600mm². The skilled person will appreciate that the window 90 may be enlarged by any suitable means, for example by cutting the adhesive skin contact layer 200. The window 90 in the adhesive skin contact layer 200 enables the wound of a patient to contact, or be in fluid communication with, other layers of the wound dressing 10, for example the wound contact layer 40, when the wound dressing 10 is applied to the skin of a patient.

The adhesive skin contact layer 200 comprises an upper surface 200b and a lower surface 200a. The lower surface 200a contacts the dermal surface in use. In use, the lower surface 200a of the adhesive skin contact layer 200 is detachably adhered to a dermal surface (not shown) of a patient such that the wound site (not shown) is located within the window 90. As such, the adhesive skin contact layer 200 is not in direct contact with the wound site but does wholly surround it. Thus, the adhesive skin contact layer 200 is in contact with intact peri-wound skin only. The upper surface 200b may be outwardly facing away from the patient's body.

A removable cover or "release layer" 21 may be adhered to the lower surface 200a of the adhesive skin contact layer 200. The removable cover 21, which may comprise folded grip sections 21a, is removable from the lower surface 200a of the adhesive skin contact layer 200. Thus, the removable cover protects the adhesive skin contact layer 200 when the removable cover 21 is adhered to the adhesive skin contact layer 200, but when the removable cover 21 is removed from the lower surface 200a of the adhesive skin contact layer 200 for use of the wound dressing 10, the lower surface 200a is exposed and able to releasably secure the wound dressing 10 to the skin of a patient.

The airway 80 may comprise a connector 82 at its distal end. The connector 82 may connect the airway 80 to an internal lumen of a conduit or tube (not shown) which in turn is connected to a source of negative pressure. The source of negative pressure may comprise a pump (not shown) for generating negative pressure and the conduit or tube may connect the pump and wound dressing 10 via the airway 80. The pump may provide negative pressure to the wound dressing 10 via the internal lumen of the conduit or tube, along the airway 80, and through an opening 12 in the wound dressing 10. In the present embodiment, the source of negative pressure may comprise a one-way valve (not shown) in-line between the pump and the wound dressing 1 to maintain a negative pressure within the wound dressing 1 when the pump is disconnected from the tube.

The airway may further comprise an adhesive layer 81 at its proximal end for adhering the proximal end of the airway 80 to a second surface 7b0 (outer facing surface) of the backing layer 70. The adhesive layer 81 may comprise an opening 12a for allowing a flow of air from the wound dressing 10 into the airway 80.

The opening 12 may comprise an opening in each of the backing layer 70 and absorbent structure 11. The backing layer 70 may comprise an opening 12b, and the absorbent structure 11 may comprise an opening 12c. Openings 12b and 12c may be arranged off-centre relative to the periphery of the backing layer 70 and the absorbent structure 11, respectively. Opening 12c may comprise corresponding openings in each of the superabsorbent layer 60, transmission layer 50 and wound contact layer 40. Opening 12c may be in fluid communication with the window 90 and, therefore, the wound site. The openings 12a, 12b and 12c may provide a fluid pathway between the wound site and the airway 80. Thus, in use, the openings 12a, 12b and 12c allow for the flow of air from the wound site, through the absorbent structure 11, through the backing layer 70, along the airway 80 and along a tube or conduit (not shown) toward a source of negative pressure (not shown).

The backing layer 70 may comprise a first surface 7a0 and the second surface 7b0. In use, the first surface 7a0 may be inwardly facing toward the patient's body, and the second surface 7b0 may be arranged opposite the first surface 7a0, outwardly facing away from the patient's body.

The absorbent structure 11 may comprise a first surface and a second surface. In use, the first surface of the absorbent structure 11 may predominantly comprise a wound facing surface of the wound contact layer 40. In use, the second surface of the absorbent structure 11 may predominantly comprise an outwardly facing surface of the superabsorbent layer 60. The first surface of the absorbent structure 11 may be adjacent, and in contact with, the upper surface 200b of the adhesive skin contact layer 200. In particular, the peripheral portion of the first surface of the absorbent structure 11 may be in contact with the upper surface 200b of the adhesive skin contact layer 200. The peripheral edge of the second surface of the absorbent structure 11 may be adjacent, and in contact with, the first surface 7a0 of the backing layer 70.

The wound contact layer 40 may comprise a first surface arranged opposite a second surface. In use, the first surface may be inwardly facing toward a patient's body, and the second surface may be outwardly facing, away from the patient's body. The first surface of the wound contact layer 40 may contact the wound when the wound dressing 10 is adhered to the skin adjacent to the wound (i.e., when in use). The first surface of the wound contact layer 40 may be substantially aligned with the window 90 of the adhesive skin contact layer 200. As such, in use, the first surface contacts the wound through the window 90. The second surface of the wound contact layer 40 may be adjacent, and in contact with, a first surface of the transmission layer 50 or a first surface of the superabsorbent layer 60. In the described embodiment, the second surface of the wound contact layer 40 is adjacent, and in contact with, the first surface of the transmission layer 50.

The transmission layer 50 may comprise a first surface arranged opposite a second surface. In use, the first surface may be inwardly facing toward a patient's body, and the second surface may be outwardly facing away from the patient's body. The first surface of the transmission layer 50 may be adjacent, and in contact with, the second surface of the wound contact layer 40 or a second surface of the superabsorbent layer 60. The second surface of the transmission layer 50 may be adjacent, and in contact with, a first surface of the superabsorbent layer 60 or the first surface 7a of the backing layer 70. In the described embodiment, the first surface of the transmission layer 50 is adjacent, and in contact with, the second surface of the wound contact layer 40, and the second surface of the transmission layer 50 is adjacent, and in contact with, the first surface of the superabsorbent layer 60.

The superabsorbent layer 60 may comprise a first surface arranged opposite a second surface. In use, the first surface may be inwardly facing toward a patient's body, and the second surface may be outwardly facing away from the patient's body. The first surface of the superabsorbent layer 60 may be adjacent, and in contact with, the second surface of the wound contact layer 40 or the second surface of the transmission layer 50. The second surface of the superabsorbent layer 60 may be adjacent, and in contact with, the first surface of the transmission layer 50 or the first surface 7a of the backing layer 70. In the described embodiment, the first surface of the superabsorbent layer 60 is adjacent, and in contact with, the second surface of the transmission layer 50, and the second surface of the superabsorbent layer 60 is adjacent, and in contact with, the first surface 7a of the backing layer 70.

The adhesive skin contact layer 200 may radially overlap the absorbent structure 11. The adhesive skin contact layer 200 may comprise an interior portion adjacent to and surrounding the window 90, and an exterior portion radially outwardly from the interior portion. The interior portion and the exterior portion of the first surface 20a of the adhesive skin contact layer 200 may contact and adhere to the skin of a patient in use. The interior portion of the second surface 200b of the adhesive skin contact layer 200 may be adjacent, and in contact with, a portion of the first surface of the wound contact layer 40. The exterior portion of the second surface 200b of the adhesive skin contact layer 200 may be adjacent, and joined with, a peripheral portion of the first surface 7a0 of the backing layer 70. Beneficially, this helps to prevent wound exudate escaping from the confines of the wound dressing which would be unhygienic and cause discomfort for the user. Moreover, this also helps maintain a negative pressure at the wound site.

The exterior portion of the second surface 200b of the adhesive skin contact layer 200 may be joined to the peripheral portion of the first surface 7a0 of the backing layer 70 by heat lamination, adhesive, welding or stitching.

The exterior portion of the second surface 200b of the adhesive skin contact layer 200 may extend radially beyond the periphery of the absorbent structure by about 27.5 mm.

At least two of the wound contact layer 40, the transmission layer 50 and the superabsorbent layer 60 may be laminated together. In the described embodiment, each of the wound contact layer 40, the transmission layer 50 and the superabsorbent layer 60 are laminated together, therefore forming a consolidated absorbent structure 11. Advantageously, this means that the absorbent structure 11 of the present invention is manufactured without requiring any relatively rigid dressing and binding components. Thus, the present invention provides an absorbent structure 11 which is flexible, therefore benefits user comfort, and which is cheaper to manufacture than absorbent structures and, therefore negative pressure wound dressings, of the prior art. Moreover, beneficially lamination does not require any complex equipment or manufacturing techniques. Thus, the present invention is simple to manufacture.

Each of the wound contact layer 40, the transmission layer 50 and the superabsorbent layer 60 may be laminated together by a scatter-coat adhesive. The scatter-coat adhesive may be a hot melt, scatter-coat adhesive. In the described embodiment, the scatter-coat adhesive is polycaprolactone and has the following properties:
- a melt flow index of between 5.2 g/10min and 11.3 g/10min, as tested with 2.16 kg, 1" PVC die at 160°C;
- a maximum water content of 0.35%;
- a mean molecular weight of 50,000;
- a melting point of 60°C; and
- a solubility parameter of 9.34 cal/cm³ to 9.43 cal/cm³.

The scatter-coat adhesive may comprise particles of which at least 98% have a particle size of 0.6mm.

The wound contact layer 40, the transmission layer 50 and the superabsorbent layer 60 may be laminated together by a laminator having the following processing properties:
- Heating zone temperature of 125°C;
- Cooling zone temperature of 25°C;
- Web tension (for each unwind reel) of 125 N;
- Crush roller pressure of 1000 N; and
- Web speed of 10 m/min.

The wound contact layer 40 may further comprise a central region and a peripheral region. The central region may be substantially square and is bordered on each side by the peripheral region.

In the described embodiment, the first surface, which contacts the wound in use, of the wound contact layer 40 may comprise warp stitches (longitudinal to the plane of the wound contact layer 40) only, i.e., the first surface does not comprise any weft stitches (transverse to the plane of the wound contact layer 40). Advantageously, this means that the amount of non-gelling fibres (which may include weft stitches) at the wound contact surface is minimised. Thus, there is a greater ratio of gelling fibres to non-gelling fibres at the wound contact surface in contact with the wound and, therefore, wound exudate. Thus, the absorption capacity of the wound contact layer is increased. As such, pooling of wound exudate at the wound site is prevented, or at least significantly reduced and an environment optimised for wound healing is maintained at the wound site.

The warp stitches of the first surface may have a pitch of 6.6mm and a stitch density of 7.5 stitches /cm.

The second surface of the transmission layer 50, which is laminated to the first surface of the transmission layer 50, comprises warp stitches and weft stitches. Each of the warp stitches and the weft stitches of the second surface of the transmission layer have a pitch of 6.6mm and a stitch density of 7.5 stitches /cm.

In other embodiments, the central region of the first surface of the wound contact layer 40 may comprise warp stitches only and the peripheral region of the first surface of the wound contact layer 40 may comprise warp and weft stitches.

The wound contact layer 40 may have a weight of 100gsm and a thickness of 3.0 mm.

Between the first surface of the wound contact layer 40 and the second surface pf the wound contact layer 40, which are joined about their periphery, the wound contact layer 40 may comprise a plurality of gel-forming fibres which, upon the uptake of wound exudate, become moist, slippery or gelatinous. Specifically, in this embodiment, the gel-forming fibres are hygroscopic fibres which upon the uptake of wound exudate become gelatinous. The gel-forming fibres may be of the type which retain their structural integrity on absorption of exudate. In the present embodiment, the fibres are sodium carboxymethylcellulose fibres having a degree of substitution of 0.30 carboxymethyl groups per glucose unit, as measured by IR spectroscopy (as defined in WO00/01425). The absence of weft stitches at the first surface of the wound contact layer 40 means that a greater amount of gel-forming fibres, as described above, are exposed at the first surface and, therefore, in contact with the wound. As such, the wound contact layer 40 is higher gelling, i.e., has a significantly greater absorbency capacity and is more retentive, than gel-forming fibres of the prior art. As such, the wound contact layer 40 absorbs a greater amount of wound exudate. Thus, pooling of wound exudate at the wound site is prevented, or at least significantly reduced.

The sodium carboxymethylcellulose fibres may be made by carboxymethylating a non-woven cellulosic fabric having an absorbency of 18g/g of Solution A (sodium/calcium chloride solution), as measured by the method described in BS EN 13726-1 (2002) "Test methods for primary wound dressings", section 3.2 "Free swell absorptive capacity". The carboxymethylation is performed by contacting the fabric with an alkali and a carboxymethylating agent such as chloroacetic acid in a combined alcohol and aqueous system. The cellulosic fabric comprises solely of cellulosic fibre and comprises continuous filament yarn.

The gel-forming fibres may have an absorbency of 2 grams 0.9% saline solution per gram of fibre (as measured by the free swell method).

The wound contact layer 40 may be stitched using lyocell yarn (produced under the trade name TencelTM yarn).

The transmission layer 50 is substantially square, as is the wound contact layer 40 and the superabsorbent layer 60, and has a thickness of 1.8mm and is made of a polyurethane foam. The transmission layer 50 may have a density of between 26 Kg/M³ and 28 Kg/M³ and a tensile strength of 150 kPa. The transmission layer 50 may have an elongation at break value of 300% and a cell count of between 17 and 26 /cm. Moreover, the transmission layer 50 may have a 40% CLD hardness of between 2.5 and 4.5 kPa.

The superabsorbent layer 60 comprises an upper layer having the second surface, and a lower layer having the first surface. Between the upper layer and the lower layer, there is enclosed a first material comprising non-woven fibres, an absorbent material and a hot melt binder.

The hot melt binder may be present in an amount of 70 g/m². The hot melt binder may be a copolymer and, in the described embodiment, may be ethylene-vinyl acetate (EVA). In manufacture of the superabsorbent layer, the superabsorbent layer may be heated to melt the hot melt binder and bind the non-woven fibres of the first material together. Advantageously, this prevents, or at least significantly limits, the amount of shedding of the fibres of the superabsorbent layer when in use. Thus, the presence of the hot melt binder in the superabsorbent layer significantly increases the strength of the layer, preventing dissociation of the absorbent structure.

The absorbent material may be a superabsorbent material, capable of absorbing wound exudate while allowing the passage of fluid through it. The superabsorbent material may be sodium polyacrylate and in the form of a powder which, advantageously, increases the surface area to volume ratio of the superabsorbent material, therefore enhancing the absorbent property of the material.

The non-woven fibres may be cellulose fibres. The cellulose non-woven fibres may be arranged as a cellulosic non-woven matrix of fibres and may be a support onto which the absorbent material may be incorporated, for example, in the described embodiment, the absorbent material is dispersed upon the cellulosic non-woven matrix. The absorbent material may be dispersed evenly, or substantially evenly, throughout or upon the non-woven fibres, for example the cellulosic non-woven matrix.

The upper layer and the lower layer of the superabsorbent layer 60 may each be a cellulosic fabric layer. The upper fabric layer and the lower fabric layer may be joined around their entire periphery to enclose the first material.

The superabsorbent layer 60 may comprise a plurality of fenestrations (not shown). Each of the plurality of fenestrations may be openings in the form of slits in the superabsorbent layer 60. Each fenestration may have a length equal to 80% of the length of the superabsorbent layer 60. Each of the upper layer and the lower layer may comprise a plurality of fenestrations. The fenestrations may be arranged in a direction planar to a longitudinal axis and transverse to a longitudinal axis of the superabsorbent layer 60. Advantageously, this arrangement aids in managing a flow of exudate through the superabsorbent layer 60 of the wound dressing 10. The fenestrations encourage or enable wound exudate to travel laterally on the superabsorbent layer 60, as opposed to axially, and assist in negative pressure transmission through the superabsorbent layer 60.

The superabsorbent layer 60 may have a basis weight of 460 g/m², a thickness of 2.55 mm and a density of 0.255 g/cm³. Moreover, the superabsorbent layer 60 may have a tensile strength (dry) of 45 N/50mm, an absorbent capacity of 25 g/g 10min, and an absorbency under compression capacity of 0.85 g/cm².

Each of the wound contact layer 40, transmission layer 50 and superabsorbent layer 60 are substantially planar.

In use, the wound dressing 10 forms part of a negative pressure wound exudate management system comprising the wound dressing 10 and further comprising a source of negative pressure (not shown) and a coupling member (for example, airway 80) for providing negative pressure to the wound dressing from an internal lumen of a conduit or tube (not shown) and through an opening 12 in the wound dressing 10. The source of negative pressure comprises a pump for generating negative pressure and the tube connects the pump and wound dressing 10 via the airway 80. In the present embodiment, the source of negative pressure may comprise a one-way valve (not shown) in-line between the pump and the wound dressing 10 to maintain a negative pressure within the wound dressing 10 when the pump is disconnected from the tube.

In use, the folded grip sections 21a of the removable cover 21 are separated and peeled in opposite directions to remove the removable cover 21 from the lower surface 200a of the adhesive skin contact layer 200. The adhesive skin contact layer 200 is therefore exposed and is then adhered to the intact peri-wound skin around a wound. The wound is located within the window 90 of the adhesive skin contact layer 200. The wound dressing 10 is connected to a source of negative pressure before or after application of the dressing to the wound. Upon leaving the wound, wound exudate contacts the first surface of the wound contact layer 40 and wicks away from the wound site, toward the transmission layer 50. The high gelling ability of the wound contact layer 40 facilitates absorption and retention of the wound exudate. Lamination of the wound contact layer 40, transmission layer 50 and superabsorbent layer 60 means each of these layers form a consolidated, absorbent and retentive structure 11. The wound exudate wicks from the upper surface of the wound contact layer 40 to the transmission layer 50. Lateral movement of the exudate occurs in the transmission layer 50 to spread the wound exudate across the surface area of the dressing. The exudate then wicks to the superabsorbent layer 60 where it is absorbed and retained prior to being transmitted out of the dressing 10 via vapour transmission through the backing film 70.

The one or more embodiments are described above by way of example only. Many variations are possible without departing from the scope of protection afforded by the appended claims.

## Claims

1. A negative pressure wound dressing (1, 10), the dressing comprising a backing layer (7, 70), an adhesive skin contact layer (2, 20, 200) and an absorbent structure (11) arranged between the backing layer (7, 70) and the adhesive skin contact layer (2, 20, 200), wherein the adhesive skin contact layer (2, 20, 200) is configured to detachably adhere the dressing to a dermal surface, wherein the backing layer (7, 70) comprises a coupling member (8, 80) configured to connect the dressing (1, 10) to a negative pressure source, wherein the adhesive skin contact layer (2, 20, 200) has a weight of between 40gsm and 250gsm, further wherein the adhesive skin contact layer (2, 20, 200) is a perforated mesh comprising perforations (3, 30) each having a diameter of between 1.25mm and 5.00mm.

2. A negative pressure wound dressing (1, 10) according to claim 1, wherein the adhesive skin contact layer (2, 20, 200) has a weight of between 175gsm and 225gsm.

3. A negative pressure wound dressing (1, 10) according to claim 1 or 2, wherein the adhesive skin contact layer (2, 20, 200) has a weight of 200gsm.

4. A negative pressure wound dressing (1, 10) according to any of claims 1 to 3, wherein the perforations (3, 30) each have a diameter of between 2.25mm and 5.00mm.

5. A negative pressure wound dressing (1, 10) according to any preceding claim, wherein the adhesive skin contact layer (2, 20, 200) comprises at least two perforations (3, 30) having different diameters.

6. A negative pressure wound dressing (1, 10) according to any preceding claim, wherein the perforations (3, 30) are arranged in a square-packed layout or a triangle packed layout.

7. A negative pressure wound dressing (1, 10) according to any preceding claim, wherein each perforation (3, 30) comprises an opening comprising a 2D shape selected from the group comprising: a circle, an oval, a triangle, a square, a rectangle, a hexagon or an octagon.

8. A negative pressure wound dressing according to any preceding claim, wherein the perforations (3, 30) have a pitch in a first direction of between 6.0mm and 10.0mm, or between 6.5mm and 10.0mm, or of 8.0mm.

9. A negative pressure wound dressing according to any preceding claim, wherein the perforations (3, 30) have a pitch in a second direction of between 4.0mm and 7.0mm, or of 5.6mm.

10. A negative pressure wound dressing according to any preceding claim, wherein the perforations (3, 30) have a total area of between 1300mm² and 2500mm², or of 2100mm².

11. A negative pressure wound dressing according to any preceding claim, wherein the degree of perforation is between 5% and 50% of the area of the adhesive skin contact layer (2, 20, 200).

12. A negative pressure wound dressing according to any preceding claim, wherein the adhesive skin contact layer (2, 20, 200) has a thickness of between 0.5mm and 1.5mm.

13. A negative pressure wound dressing according to any preceding claim, wherein the width and length of the adhesive skin contact layer (2, 20, 200) are each between 100mm and 160mm, or are each 130mm.

14. A negative pressure wound dressing according to any preceding claim, wherein the perforations (3, 30) are formed by punch perforating, a hot pin process, a laser ablation process or an ultrasound process.

15. A method for forming a plurality of perforations (3, 30) in the adhesive skin contact layer (2, 20, 200) of the negative pressure wound dressing (1, 10) according to any preceding claim, the method comprising:
a. Providing an adhesive skin contact layer; and
b. Forming a plurality of perforations in the adhesive skin contact layer by punch perforating, wherein the adhesive skin contact layer has a weight of between 40gsm and 250gsm, further wherein the adhesive skin contact layer is a perforated mesh comprising perforations each having a diameter of between 1.25mm and 5.00mm.

## Patentansprüche

1. Unterdruck-Wundverband (1, 10), wobei der Verband eine Rückschicht (7, 70), eine klebende Hautkontaktschicht (2, 20, 200) und eine Absorptionsstruktur (11), angeordnet zwischen der Rückschicht (7, 70) und der klebenden Hautkontaktschicht (2, 20, 200), umfasst, wobei die klebende Hautkontaktschicht (2, 20, 200) ausgebildet ist, um den Verband lösbar an eine Hautoberfläche zu kleben, wobei die Rückschicht (7, 70) ein Kopplungselement (8, 80) umfasst, ausgebildet, um den Verband (1, 10) mit einer Unterdruckquelle zu verbinden, wobei die klebende Hautkontaktschicht (2, 20, 200) ein Gewicht zwischen 40 g/m² und 250 g/m² aufweist, wobei ferner die klebende Hautkontaktschicht (2, 20, 200) ein perforiertes Netz, umfassend Perforationen (3, 30), jeweils aufweisend einen Durchmesser zwischen 1,25 mm und 5,00 mm, ist.

2. Unterdruck-Wundverband (1, 10) gemäß Anspruch 1, wobei die klebende Hautkontaktschicht (2, 20, 200) ein Gewicht zwischen 175 g/m² und 225 g/m² aufweist.

3. Unterdruck-Wundverband (1, 10) gemäß Anspruch 1 oder 2, wobei die klebende Hautkontaktschicht (2, 20, 200) ein Gewicht von 200 g/m² aufweist.

4. Unterdruck-Wundverband (1, 10) gemäß einem der Ansprüche 1 bis 3, wobei die Perforationen (3, 30) jeweils einen Durchmesser zwischen 2,25 mm und 5,00 mm aufweisen.

5. Unterdruck-Wundverband (1, 10) gemäß einem vorhergehenden Anspruch, wobei die klebende Hautkontaktschicht (2, 20, 200) mindestens zwei Perforationen (3, 30), aufweisend unterschiedliche Durchmesser, umfasst.

6. Unterdruck-Wundverband (1, 10) gemäß einem vorhergehenden Anspruch, wobei die Perforationen (3, 30) in einer quadratisch gepackten Anordnung oder einer dreieckig gepackten Anordnung angeordnet sind.

7. Unterdruck-Wundverband (1, 10) gemäß einem vorhergehenden Anspruch, wobei jede Perforation (3, 30) eine Öffnung, umfassend eine 2D Form, ausgewählt aus der Gruppe, umfassend: einen Kreis, ein Oval, ein Dreieck, ein Quadrat, ein Rechteck, ein Sechseck oder ein Achteck, umfasst.

8. Unterdruck-Wundverband gemäß einem vorhergehenden Anspruch, wobei die Perforationen (3, 30) eine Teilung in einer ersten Richtung zwischen 6,0 mm und 10,0 mm oder zwischen 6,5 mm und 10,0 mm oder von 8,0 mm aufweisen.

9. Unterdruck-Wundverband gemäß einem vorhergehenden Anspruch, wobei die Perforationen (3, 30) eine Teilung in einer zweiten Richtung zwischen 4,0 mm und 7,0 mm oder von 5,6 mm aufweisen.

10. Unterdruck-Wundverband gemäß einem vorhergehenden Anspruch, wobei die Perforationen (3, 30) eine Gesamtfläche zwischen 1300 mm² und 2500 mm² oder von 2100 mm² aufweisen.

11. Unterdruck-Wundverband gemäß einem vorhergehenden Anspruch, wobei der Perforationsgrad zwischen 5 % und 50 % der Fläche der klebenden Hautkontaktschicht (2, 20, 200) ist.

12. Unterdruck-Wundverband gemäß einem vorhergehenden Anspruch, wobei die klebende Hautkontaktschicht (2, 20, 200) eine Dicke zwischen 0,5 mm und 1,5 mm aufweist.

13. Unterdruck-Wundverband gemäß einem vorhergehenden Anspruch, wobei die Breite und die Länge der klebenden Hautkontaktschicht (2, 20, 200) jeweils zwischen 100 mm und 160 mm liegen oder jeweils 130 mm ist.

14. Unterdruck-Wundverband gemäß einem vorhergehenden Anspruch, wobei die Perforationen (3, 30) durch Stanzperforieren, ein Heißstiftverfahren, ein Laserablationsverfahren oder ein Ultraschallverfahren ausgebildet sind.

15. Verfahren zum Ausbilden einer Mehrzahl von Perforationen (3, 30) in der klebenden Hautkontaktschicht (2, 20, 200) des Unterdruck-Wundverbands (1, 10) gemäß einem vorhergehenden Anspruch, wobei das Verfahren umfasst:
a. Bereitstellen einer klebenden Hautkontaktschicht; und
b. Ausbilden einer Mehrzahl von Perforationen in der klebenden Hautkontaktschicht durch Stanzperforieren, wobei die klebende Hautkontaktschicht ein Gewicht zwischen 40 g/m² und 250 g/m² aufweist, wobei ferner die klebende Hautkontaktschicht ein perforiertes Netz, umfassend Perforationen, jeweils aufweisend einen Durchmesser zwischen 1,25 mm und 5,00 mm, ist.

## Revendications

1. Un pansement (1, 10) à dépression pour une plaie, le pansement comprenant une couche (7, 70) de support, une couche (2, 20, 200) adhésive de contact avec la peau et une structure (11) absorbante disposée entre la couche (7, 70) de support et la couche (2, 20, 200) adhésive de contact avec la peau, dans lequel la couche (2, 20, 200) adhésive de contact avec la peau est configurée pour faire adhérer de manière détachable le pansement a une surface dermique, dans lequel la couche (7, 70) de support comprend un élément (8, 80) de communication configuré pour mettre le pansement (1, 10) en communication avec une source de dépression, dans lequel la couche (2, 20, 200) adhésive de contact avec la peau a un poids compris entre 40 gm² et 250 gm², dans lequel en outre la couche (2, 20, 200) adhésive de contact avec la peau est un tamis perforé comprenant des perforations (3, 30) ayant chacune un diamètre compris entre 1,25 mm et 5,00 mm.

2. Un pansement (1, 10) à dépression pour une plaie suivant la revendication 1, dans lequel la couche (2, 20, 200) adhésive de contact avec la peau a un poids compris entre 175 gm² et 225 gm².

3. Un pansement (1, 10) à dépression pour une plaie suivant la revendication 1 ou 2, dans lequel le couche (2, 20, 200) adhésive de contact avec la peau a un poids de 200 gm².

4. Un pansement (1, 10) à dépression pour une plaie suivant l'une quelconque des revendications 1 à 3, dans lequel les . perforations (3, 30) ont chacune un diamètre compris entre 2,25 mm et 5,00 mm.

5. Un pansement (1, 10) à dépression pour une plaie suivant l'une quelconque des revendications précédentes, dans lequel la couche (2, 20, 200) adhésive de contact avec la peau comprend au moins deux perforations (3, 30) ayant des diamètres différents.

6. Un pansement (1, 10) à dépression pour une plaie suivant l'une quelconque des revendications précédentes, dans lequel les perforations (3, 30) sont disposées suivant une configuration en carré ou une configuration en triangle.

7. Un pansement (1, 10) à dépression pour une plaie suivant l'une quelconque des revendications précédentes, dans lequel chaque perforation (3, 30) comprend une ouverture comprenant une forme en 2D choisie dans le groupe comprenant un cercle, un ovale, un triangle, un carré, un rectangle, un hexagone ou un octogone.

8. Un pansement à dépression pour une plaie suivant l'une quelconque des revendications précédentes, dans lequel les perforations (3, 30) sont à une distance dans une première direction comprise entre 6,0 mm et 10,0 mm ou entre 6,5 mm et 10 mm ou de 8,0 mm.

9. Un pansement à dépression pour une plaie suivant l'une quelconque des revendications précédentes, dans lequel lesdites perforations (3, 30) sont à une distance dans une deuxième direction comprise entre 4,0 mm et 7,0 mm ou de 5,6 mm.

10. Un pansement à dépression pour une plaie suivant l'une quelconque des revendications précédentes, dans lequel les perforations (3, 30) ont une surface totale comprise entre 1300 mm² et 2500 mm² ou de 2100 mm².

11. Un pansement à dépression pour une plaie suivant l'une quelconque des revendications précédentes, dans lequel le degré de perforation représente de 5 % à 50 % de la surface de la couche (2, 20, 200) adhésive de contact avec la peau.

12. Un pansement à dépression pour une plaie suivant l'une quelconque des revendications précédentes, dans lequel la couche (2, 20, 200) adhésive de contact avec la peau a une épaisseur comprise entre 0,5 mm et 1,5 mm.

13. Un pansement à dépression pour une plaie suivant l'une quelconque des revendications précédentes, dans lequel la largeur et la longueur de la couche (2, 20, 200) adhésive de contact avec la peau sont chacune comprises entre 100 mm et 160 mm ou sont chacune de 130 mm.

14. Un pansement à dépression pour une plaie suivant l'une quelconque des revendications précédentes, dans lequel les perforations (3, 30) sont formées par poinçonnage, par un procédé à broche chaude, par une opération par ablation laser ou par une opération par ultrasons.

15. Un procédé de formation d'une pluralité de perforations (3, 30) dans la couche (2, 20, 200) adhésive de contact avec la peau du pansement (1, 10) à dépression pour une plaie suivant l'une quelconque des revendications précédentes, le procédé comprenant :
a. se procurer une couche adhésive de contact avec la peau ; et .
b. former une pluralité de perforations dans la couche adhésive de contact avec la peau par poinçonnage, dans lequel la couche adhésive de contact avec la peau a un poids compris entre 40 gm² et 250 gm², dans lequel en outre la couche adhésive de contact avec la peau est un tamis perforé comprenant des perforations ayant chacune un diamètre compris entre 1,25 mm et 5,00 mm. .
